(19) Europäisches Patentamt
European Patent Office
Office européen des brevets

(11) Publication number: **0 062 321**
**B1**

(12) **EUROPEAN PATENT SPECIFICATION**

(45) Date of publication of patent specification: **01.03.89**

(51) Int. Cl.$^4$: **C 07 D 501/46,** A 61 K 31/545

(21) Application number: **82102818.0**

(22) Date of filing: **02.04.82**

(54) **New cephem compounds, processes for their preparation, pharmaceutical compositions containing them and their starting compounds.**

(30) Priority: **03.04.81 GB 8110556**
**24.07.81 GB 8122896**
**30.10.81 GB 8132775**

(43) Date of publication of application:
**13.10.82 Bulletin 82/41**

(45) Publication of the grant of the patent:
**01.03.89 Bulletin 89/09**

(84) Designated Contracting States:
**AT BE CH DE FR GB IT LI LU NL SE**

(56) References cited:
**EP-A-0 025 017**
**EP-A-0 047 977**
**FR-A-2 439 786**
**FR-A-2 441 630**
**FR-A-2 449 692**

**The file contains technical information submitted after the application was filed and not included in this specification**

(73) Proprietor: **FUJISAWA PHARMACEUTICAL CO., LTD.**
**3, Doshomachi 4-chome Higashi-ku**
**Osaka-shi Osaka 541 (JP)**

(72) Inventor: **Teraji, Tsutomu**
**No. 6-20-6, Kofudai Toyono-cho**
**Toyono-gun Osaka-fu (JP)**
Inventor: **Sakane, Kazuo**
**No. 6-60-5, Higashi-sonoda-cho**
**Amagasaki (JP)**
Inventor: **Goto, Jiro**
**A-401 Senridai-sukai-taun No. 21**
**Kashikiriyama, Suita (JP)**

(74) Representative: **Türk, Gille, Hrabal**
**Bruckner Strasse 20**
**D-4000 Düsseldorf 13 (DE)**

**EP 0 062 321 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to new cephem compounds and pharmaceutically acceptable salts thereof. More particularly, it relates to new cephem compounds and pharmaceutically acceptable salts thereof, which have antimicrobial activities and to processes for preparation thereof, to pharmaceutical composition comprising the same, and to a method of using the same therapeutically in the treatment of infectious diseases in human beings and animals.

EP—A2—47 977, which was published within the priority dates of the present compounds, discloses cephalosporin compounds, which in view of the proviso in the present claims, differ from the present ones with respect to the 3-substituent, which can be pyrazoliomethyl, triazoliomethyl or pyridinomethyl, all optionally substituted.

EP—A1—25 017 describes cephalosporin antibiotics, which differ from the present compounds with respect to the 3-substituent, which can be substituted pyridiniomethyl.

In FR—A—2 449 692, FR—A—2 439 786 and FR—A—2 441 630 cephalosporin antibiotics are disclosed having as 3-substituents some of those of the present compounds and distinguishing from the present ones by a thiazol ring in the 7-side chain.

It is one object of the present invention to provide new cephem compounds and pharmaceutically acceptable salts thereof, which are active against a number of pathogenic microorganisms.

Another object of the present invention is to provide processes for the preparation of new cephem compounds and pharmaceutically acceptable salts thereof.

A further object of the present invention is to provide pharmaceutical composition comprising, as active ingredients, said new cephem compounds and pharmaceutically acceptable salts thereof.

The object new cephem compounds are novel and can be represented by the following general formula (I).

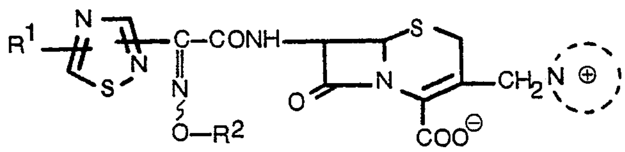

(I)

wherein

$R^1$ is amino or a protected amino group,

$R^2$ is $(C_{1-6})$alkyl, $(C_{2-6})$alkenyl or $(C_{2-6})$alkynyl, each of which may be substituted with 1 to 3 substituents selected from $(C_{1-6})$alkylthio or carboxy, or cyclo$(C_{3-6})$alkenyl, and the group of the formula:

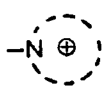

is triazolio, imidazolio, pyrimidinio, pyrazolio, pyridazinio or pyrazinio, each of which may be substituted with 1 to 3 substituents selected from $(C_{1-6})$alkyl, amino, $(C_{2-6})$alkenyl, halogen, hydroxy or hydroxy$(C_{1-6})$alkyl,

with the proviso that when $R^2$ is methyl or 2-carboxy-prop-2-yl

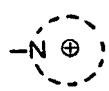

is not

$(C_{1-6})$alkyl-triazolio or $(C_{1-6})$alkyl-pyrazolio.

According to the present invention, the new cephem compounds (I) can be prepared by some processes which are illustrated in the following scheme.

*Process 1*

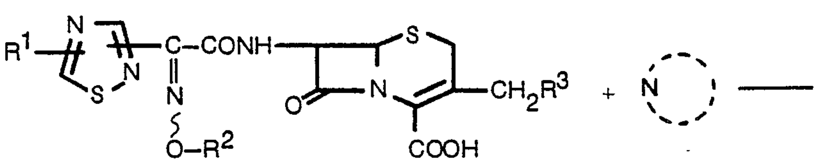

(II) (III)

or a salt thereof

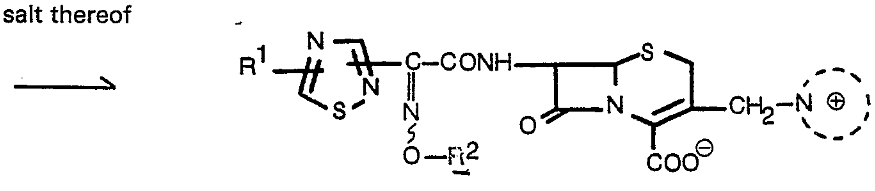

(I)

or a salt thereof

*Process 2*

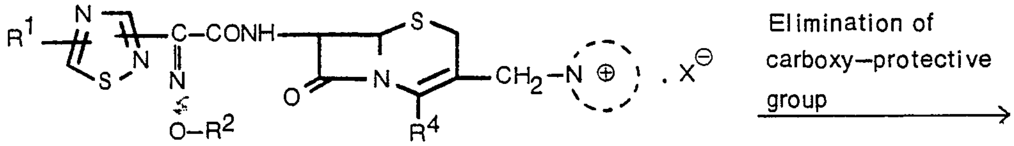


(IV)

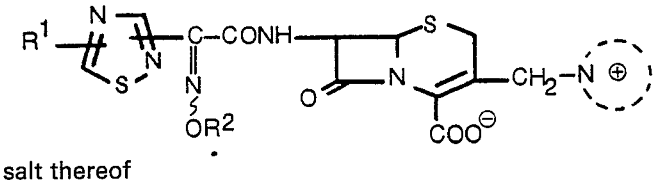

(I)

or a salt thereof

wherein

$R^1$, $R^2$ and a group of the formula:

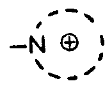

are each as defined above;

$R^3$ is a group which can be replaced by a group of the formula:

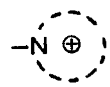 ;

a compound of the formula:

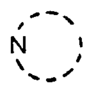

is a heterocyclic compound corresponding to

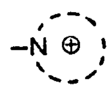 ;

$R^4$ is a protected carboxy group; and

X is an acid residue.

Among the starting compounds of the present invention, the compound (IV) is novel and can be prepared by the following methods.

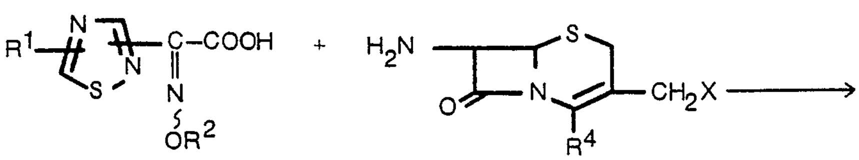

(V) (VI)

or its reactive derivative at the carboxy group or a salt thereof

or its reactive derivative at the amino group or a salt thereof

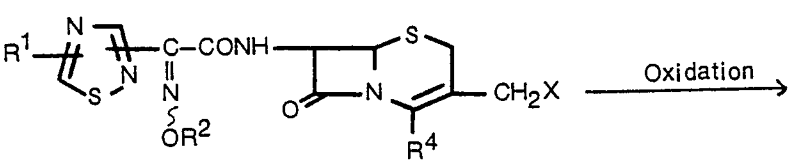


(VII)

or a salt thereof

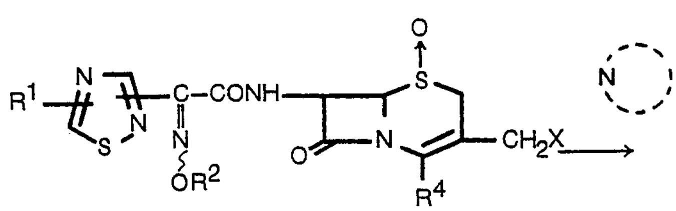

(VIII)

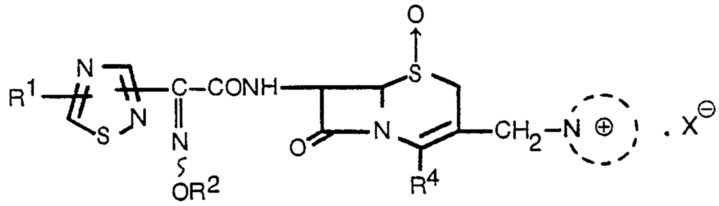

(IX)

or a salt thereof

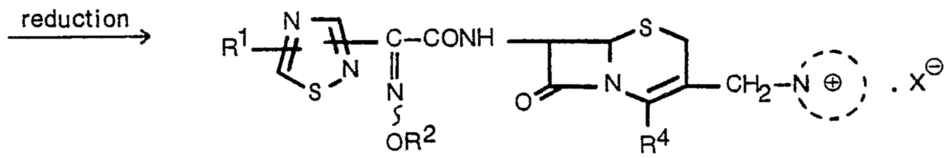


(IV)

wherein

$R^1$, $R^2$, $R^4$, X, a compound of the formula:

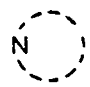

and a group of the formula:

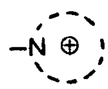

are each as defined above.

Regarding the object compound (I) and the starting compound (II), (IV), (V), (VII), (VIII) and (IX), it is to be understood that said object and starting compounds include syn isomer, anti isomer and a mixture thereof. For example, with regard to the object compounds (I), syn isomer means one geometrical isomer having the partial structure represented by the following formula:

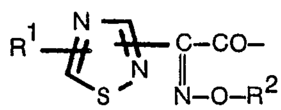

(wherein $R^1$ and $R^2$ are each as defined above) and anti isomer means the other geometrical isomer having the partial structure represented by the following formula:

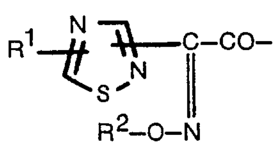

(wherein $R^1$ and $R^2$ are each as defined above).

Regarding the starting compounds (II), (IV), (V), (VII), (VIII) and (IX) as mentioned above, the syn isomer and the anti isomer can also be referred to the same geometrical isomers as illustrated for the compound (I).

Suitable pharmaceutically acceptable salts of the object compound (I) are conventional non-toxic salt and include a metal salt such as an alkali metal salt (e.g. sodium salt, potassium salt, etc.) and an alkaline earth metal salt (e.g. calcium salt, magnesium salt, etc.), an ammonium salt, an organic base salt (e.g. trimethylamine salt, triethylamine salt, pyridine salt, picoline salt, dicyclohexylamine salt, N,N'-dibenzylethylenediamine salt, etc.), an organic acid salt (e.g. acetate, maleate, tartrate, methanesulfonate, benzenesulfonate, formate, toluenesulfonate, etc.), an inorganic acid salt (e.g. hydrochloride, hydrobromide, hydroiodide, sulfate, phosphate, etc.), or a salt with an amino acid (e.g. arginine, aspartic acid, glutamic acid, etc.), and the like.

In the above and subsequent descriptions of the present specification, suitable examples and illustrations of the various definitions which the present invention include within the scope thereof are explained in details as follows.

The term "$(C_{1-6})$" is intended to mean 1 to 6 carbon atoms, unless otherwise indicated.

Suitable "protected amino" for $R^1$ may include an acylamino or an amino group substituted by a conventional protecting group such as ar$(C_{1-6})$alkyl which may have at least one suitable substituent(s) (e.g. benzyl, trityl, etc.) or the like.

Suitable acyl moiety in the terms "acylamino" and "acyloxy" may include carbamoyl, aliphatic acyl group and acyl group containing an aromatic or heterocyclic ring. And, suitable examples of the said acyl

may be $(C_{1-6})$alkanoyl (e.g. formyl, acetyl, propionyl, butyryl, isobutyryl, valeryl, isovaleryl, oxalyl, succinyl, pivaloyl, etc.);

$(C_{1-6})$alkoxycarbonyl (e.g. methoxycarbonyl, ethoxycarbonyl, propoxycarbonyl, 1-cyclopropylethoxycarbonyl, isopropoxycarbonyl, butoxycarbonyl, tertiarybutoxycarbonyl, pentyloxycarbonyl, hexyloxycarbonyl, etc.);

$(C_{1-6})$ alkanesulfonyl (e.g. mesyl, ethanesulfonyl, propanesulfonyl, isopropanesulfonyl, butanesulfonyl, etc.);

arenesulfonyl (e.g. benzenesulfonyl, tosyl, etc.); aroyl (e.g. benzoyl, toluoyl, xyloyl, naphthoyl, phthaloyl, indancarbonyl, etc.);

ar$(C_{1-6})$alkanoyl (e.g. phenylacetyl, phenylpropionyl, etc.);

ar$(C_{1-6})$alkoxycarbonyl (e.g. benzyloxycarbonyl, phenethyloxycarbonyl, etc.), and the like.

The acyl moiety as stated above may have at least one suitable substituent(s) such as acyl (e.g. $(C_{1-6})$ alkanoyl), halogen (chlorine, bromine, fluorine and iodine) or the like.

Suitable "$(C_{2-6})$" alkenyl is one having 2 to 6 carbon atoms and may include vinyl, allyl, isopropenyl, 1-propenyl, 2-butenyl, 3-pentenyl and the like, and preferably one having 2 to 4 carbon atoms.

Suitable "$(C_{2-6})$ alkynyl" is one having 2 to 6 carbon atoms and may include ethynyl, 2-propynyl, 2-butynyl, 3-pentynyl, 3-hexynyl and the like, and preferably one having 2 to 4 carbon atoms.

The $(C_{1-6})$alkyl, $(C_{2-6})$alkenyl and $(C_{2-6})$alkynyl groups as mentioned above may be substituted with 1 to 3 substituent(s) selected from carboxy $(C_{1-6})$ alkylthio (e.g. methylthio, ethylthio, propylthio, isopropylthio, butylthio, pentylthio, hexylthio, etc.).

Suitable "cyclo$(C_{3-6})$alkenyl" may include one having 3 to 6 carbon atoms, for example, cyclopentenyl, cyclohexenyl or the like, preferably one having 5 to 6 carbon atoms.

Suitable $R^3$ may include an acid residue such as acyloxy, halogen (e.g. chlorine, bromine, iodine or fluorine), azido or the like, wherein acyl moiety in the term "acyloxy" can be referred to the ones as exemplified above.

Suitable groups of the formula

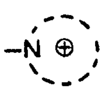

are imidazolio, pyrazolio, triazolio (e.g. 1,2,3-triazolio, 1,3,4-triazolio, or 1,2,4-triazolio), pyridazinio, pyrimidinio, or pyrazinio, may be substituted with 1 to 3 substituent(s) selected from hydroxy; $(C_{1-6})$ alkyl (e.g. methyl, ethyl, propyl, isopropyl, butyl, isobutyl, pentyl, hexyl, etc.); amino; halogen (e.g. chlorine, bromine, iodine or fluorine); $(C_{2-6})$ alkenyl as mentioned above; hydroxy$(C_{1-6})$alkyl (e.g., hydroxymethyl, hydroxyethyl, etc.).

Suitable "protected carboxy" may include "esterified carboxy" and suitable ester moiety in the term "esterified carboxy" may be the ones such as $C_{1-6}$ alkyl ester (e.g. methyl ester, ethyl ester, propyl ester, isopropyl ester, butyl ester, isobutyl ester, t-butyl ester, pentyl ester, t-pentyl ester, hexyl ester, 1-cyclopropylethyl ester, etc.); $(C_{2-6})$ alkenyl ester (e.g. vinyl ester, allyl ester, etc.); $(C_{2-6})$ alkynyl ester (e.g. ethynyl ester, propynyl ester, etc); mono (or di or tri)-halo$(C_{1-6})$alkyl ester (e.g. 2-iodoethyl ester, 2,2,2-trichloroethyl ester, etc.); $(C_{1-6})$ alkanoyloxy$(C_{1-6})$alkyl ester (e.g. acetoxymethyl ester, propionyloxymethyl ester, butyryloxymethyl ester, valeryloxymethyl ester, pivaloyloxymethyl ester, hexanoyloxymethyl ester, 2-acetoxyethyl ester, 2-propionyloxyethyl ester, etc.); $(C_{1-6})$ alkanesulfonyl$(C_{1-6})$alkyl ester (e.g. mesylmethyl ester, 2-mesylethyl ester etc.); ar$(C_{1-6})$alkyl ester, for example, phenyl$(C_{1-6})$alkyl ester which may be substituted with one or more suitable substituent(s) (e.g. benzyl ester, 4-methoxybenzyl ester, 4-nitrobenzyl ester, phenethyl ester, trityl ester, diphenylmethyl ester, bis(methoxyphenyl)methyl ester, 3,4-dimethoxybenzyl ester, 4-hydroxy-3,5-ditertiarybutylbenzyl ester, etc.); aryl ester which may have one or more suitable substituent(s) (e.g. phenyl ester, tolyl ester, tertiarybutylphenyl ester, xylyl ester, mesityl ester, cumenyl ester, etc.), and the like.

A compound of the formula

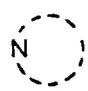

means a heterocyclic compound corresponding to a group of the formula

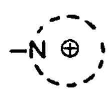

Suitable acid residue for X can be referred to the ones as stated above.

Preferred embodiments of the object compounds (I) are as follows.

Preferred embodiments of $R^1$ is amino;

$R^2$ is $(C_{1-6})$alkyl, carboxy$(C_{1-6})$alkyl, $(C_{1-6})$alkylthio$(C_{1-6})$alkyl, $(C_{2-6})$alkenyl, $(C_{2-6})$alkynyl or cyclo$(C_{3-6})$alkenyl; and

a group of the formula;

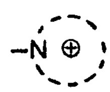

is triazolio, imidazolio, pyrimidinio, pyrazolio, pyridazinio or pyrazinio, each of which may be substituted with one $(C_{1-6})$alkyl, amino, $(C_{2-6})$alkenyl, halogen, hydroxy or hydroxy$(C_{1-6})$alkyl.

The processes for prepariing the object compounds of the present invention are explained in details in the following.

Process 1:

The object compound (I) or a salt thereof can be prepared by reacting the compound (II) or a salt thereof with the compound (III).

Suitable salt of the compound (II) can be referred to the ones exemplified for the compound (I).

The present reaction may be carried out in a solvent such as water, phosphate buffer, acetone, chloroform, acetonitrile, nitrobenzene, methylene chloride, ethylene chloride, formamide, dimethylformamide, methanol, ethanol, ether, tetrahydrofuran, dimethylsulfoxide, or any other organic solvent which does not adversely affect the reaction, preferably in ones having strong polarities. Among the solvents, hydrophilic solvents may be used in a mixture with water. The reaction is preferably carried out in around neutral medium. When the compound (II) is used in a free form, the reaction is preferably conducted in the presence of a base, for example, inorganic base such as alkali metal hydroxide, alkali metal carbonate, alkali metal bicarbonate, organic base such as trialkylamine, and the like. The reaction temperature is not critical, and the reaction is usually carried out at ambient temperature, under warming or under heating. The present reaction is preferably carried out in the presence of alkali metal halide (e.g. sodium iodide, potassium iodide, etc.), alkali metal thiocyanate (e.g. sodium thiocyanate, potassium thiocyanate, etc.) etc.

Process 2:

The compound (I) or a salt thereof can be prepared by subjecting the compound (IV) to elimination reaction of carboxy-protective group.

In the present elimination reaction, all conventional methods used in the elimination reaction of the carboxy protective group, for example, hydrolysis, reduction, elimination using Lewis acid, etc. are applicable. When the carboxy protective group is an ester, it can be eliminated by hydrolysis or elimination using Lewis acid. The hydrolysis is preferably carried out in the presence of a base or an acid. Suitable base may include an inorganic base and an organic base.

Suitable acid may include an organic acid (e.g. formic acid, acetic acid, propionic acid, etc.) and an inorganic acid (e.g. hydrochloric acid, hydrobromic acid, sulfuric acid, etc.).

The present hydrolysis is usually carried out in an organic solvent, water or a mixed solvent thereof.

The reaction temperature is not critical, and it may suitably be selected in accordance with the kind of the carboxy protective group and the elimination method.

The elimination using Lewis acid is carried out by reacting the compound (IV) with Lewis acid such as boron trihalide (e.g. boron trichloride, boron trifluoride, etc.), titanium tetrahalide (e.g. titanium tetrachloride, titanium tetrabromide, etc.), tin tetrahalide (e.g. tin tetrachloride, tin tetrabromide etc.), aluminum halide (e.g. aluminum chloride, aluminum bromide, etc.), trihaloacetic acid (e.g. trichloroacetic acid, trifluoroacetic acid, etc.) or the like. This elimination reaction is preferably carried out in the presence of cation trapping agents (e.g. anisole, phenol, etc.) and is usually carried out in a solvent such as nitroalkane (e.g. nitromethane, nitroethane, etc.), alkylene halide (e.g. methylene chloride, ethylene chloride, etc.), diethyl ether, carbon disulfide or any other solvent which does not adversely affect the reaction. These solvents may be used as a mixture thereof. The reaction temperature is not critical, and the reaction is usually carried out under cooling, at ambient temperature or under warming.

The reduction elimination can be applied preferably for elimination of the protective group such as halo$(C_{1-6})$alkyl (e.g. 2-iodoethyl, 2,2,2-trichloroethyl, etc.) ester, ar$(C_{1-6})$alkyl (e.g. benzyl, nitrobenzyl, etc.) ester or the like. The reduction method applicable for the elimination reaction may include, for example, reduction by using a combination of a metal (e.g. zinc, zinc amalgam, etc.) or a salt of chromium compound (e.g. chromous chloride, chromous acetate, etc.) and an organic or inorganic acid (e.g. acetic acid, propionic acid, hydrochloric acid, etc.); and conventional catalytic reduction in the presence of a conventional metallic catalyst (e.g. palladium on carbon, Raney nickel, etc.).

This reduction is usually carried out in a solvent such as water, alcohol (e.g. methanol, ethanol, etc.), tetrahydrofuran or any other solvent which does not adversely affect the reaction. These solvents may be used as a mixture thereof. The reaction temperature is not critical, and the reaction is usually carried out under cooling, at ambient temperature or under warming.

The Preparations of the starting compound (IV) are explained in detail in the following.

Preparation 1

The compound (IX) can be prepared by reacting the compound (VIII) or a salt thereof with the compound of the formula:

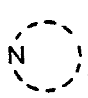.

The reaction can be carried out according to a similar manner to that of Process 1.

Preparation 2

The compound (IV) can be prepared by reducing the compound (IX).

The present reduction can be carried out in the presence of conventional reducing agent which can reduce

O
↑
—S—

group in cephalosporin compound to —S— group, for example, phosphorus halide such as phosphorus trihalide (e.g. phosphorus trichloride, etc.).

The object compounds (I) of the present invention exhibit high antimicrobial activity and inhibit the growth of a number of microorganisms including pathogenic Gram-positive and Gram-negative bacteria.

For therapeutic administration, the cephalosporin compounds according to the present invention are used in the form of pharmaceutical preparation which contain said compounds in admixture with a pharmaceutically acceptable carriers such as an organic or inorganic solid or liquid excipient suitable for oral, parenteral or external administration. The pharmaceutical preparations may be in solid form such as capsule, tablet, dragee, ointment or suppository, or in liquid form such as solution, suspension, or emulsion. If desired, there may be included in the above preparations auxiliary substances, stabilizing agents, wetting or emulsifying agents, buffers and other commonly used additives.

While the dosage of the compounds may vary from and also depend upon the age and condition of the patient, an average single dose of about 50 mg., 100 mg., 250 mg., and 500 mg. of the compounds according to the present invention has proved to be effective for treating of infectious diseases caused by a number of pathogenic bacteria. In general amounts, daily dose between 1 mg/body and about 1000 mg/body or even more may be administered.

Now in order to show the utility of the object compounds (I), test data on anti-microbial activity of representative compounds of the present invention are shown below.

Test method

One loopful of an overnight culture of each test strain in Trypticase-soy broth ($10^8$ viable cells per ml.) was streaked on heart infusion agar (HI-agar) containing graded concentrations of antibiotics, and the minimal inhibitory concentration (MIC) was expressed in terms of μg/ml after incubation at 37°C for 20 hours.

Test compounds

(1) 7-[2-Ethoxyimino-2-(5-amino-1,2,4-thiadiazol-3-yl)acetamido]-3-(2-methyl-1-pyrazoliomethyl)-3-cephem-4-carboxylate (syn isomer).

(2) 7-[2-Ethoxyimino-2-(5-amino-1,2,4-thiadiazol-3-yl)acetamido]-3-(1-pyridaziniomethyl)-3-cephem-4-carboxylate (syn isomer).

(3) 7-[2-Methoxyimino-2-(5-amino-1,2,4-thiadiazol-3-yl)acetamido]-3-(1-pyridaziniomethyl)-3-cephem-4-carboxylate (syn isomer).

Test Results

| Test Bacteria | M I C (μ/ml) Test compound (1) | (2) | (3) |
|---|---|---|---|
| Pr.vulgaris IAM—1025 | 0.78 | 0.39 | 6.25 |
| Pr.mirabilis 140 | 0.39 | 0.39 | 0.39 |

The following Preparations and Examples are given for the purpose of illustrating the present invention.

Preparation 1

To a cold solution of phosphorus pentachloride (16.6 g) in methylene chloride (150 ml) was added 2-ethoxyimino-2-(5-amino-1,2,4-thiadiazol-3-yl)acetic acid (syn isomer) (17.3 g) at −18°C and the mixture was stirred for 15 minutes at −13 to −10°C. On the other hand, a mixture of 7-amino-3-acetoacetoxymethyl-3-cephem-4-carboxylic acid (25.1 g) and trimethylsilylacetamide (80 g) in methylene chloride (400 ml) was warmed to make a clear solution and cooled to −10°C. The cold solution was added to the above activated mixture and the mixture was stirred for 1 hour at −10°C. The reaction mixture was poured into an aqueous solution (450 ml) of sodium bicarbonate (42 g) and stirred for 1 hour at room temperature. The aqueous layer was separated out, adjusted to pH 2 with 6N hydrochloric acid and extracted with ethyl acetate. The extract was washed with water, dried over anhydrous magnesium sulfate and evaporated to dryness. The residue was triturated with diethyl ether to give a powder of 7-[2-ethoxyimino-2-(5-amino-1,2,4-thiadiazol-

3-yl)acetamido]-3-acetoacetoxymethyl-3-cephem-4-carboxylic acid (syn isomer) (24.5 g), mp. 101 to 106°C (dec.).

IR (Nujol): 3400, 3300, 3170, 1770, 1710, 1660, 1620, 1525, 1145, 1035 $cm^{-1}$.

Example 1

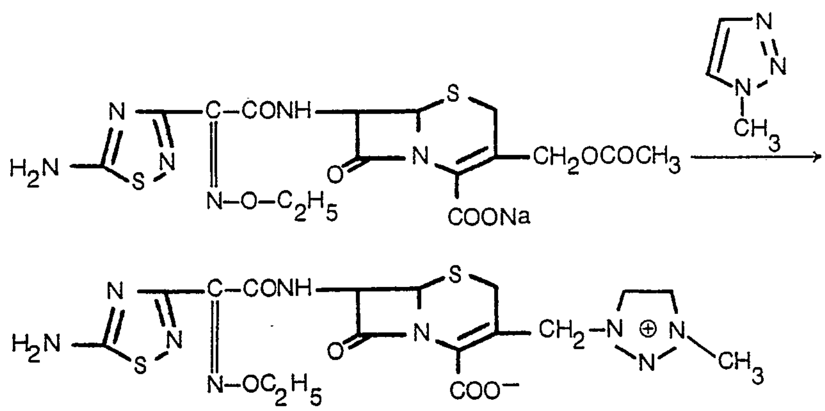

To a mixture of 1-methyl-1,2,3-triazole (2.5 ml), sodium iodide (18.0 g) and water (3.0 ml) was added sodium 7-[2-ethoxyimino-2-(5-amino-1,2,4-thiadiazol-3-yl)-acetamido]cephalosporanate (syn isomer) (5.34 g) at 80°C under stirring, which was continued for one hour at the same temperature. The mixture was cooled to room temperature, diluted with water (25 ml) and adjusted to pH 2.8 with 1N hydrochloric acid. An insoluble material was filtered off and the filtrate was washed with ethyl acetate, evaporated to remove ethyl acetate and subjected to column chromatography on a non ionic adsorption resin Diaion HP—20 (trade-mark: prepared by Mitsubishi Chemical Industries) (160 ml). After the column was washed with water, the elution was carried out with 20% aqueous methanol. The eluants containing an object compound were collected, evaporated to remove methanol under reduced pressure and lyophilized to give 7-([2-ethoxyimino-2-(5-amino-1,2,4-thiadiazol-3-yl)acetamido-3-[3-methyl-1-(1,2,3-triazolio)methyl]-3-cephem-4-carboxylate (syn isomer) (1.0 g), mp. 141 to 145°C (dec.).

IR (Nujol): 3400—3100, 1770, 1660, 1610, 1530, 1035 $cm^{-1}$.

NMR ($DMSO-d_6$, δ): 1.23 (3H, t, J=7Hz), 3.15 and 3.55 (2H, ABq, J=18Hz), 4.13 (2H, q, J=7Hz), 4.30 (3H, s), 5.00 (1H, d, J=5Hz), 5.30 and 5.59 (2H, ABq, J=14Hz), 5.67 (1H, 2d, J=5 and 8Hz), 8.16 (2H, bs), 8.82 (1H, s), 9.06 (1H, s), 9.42 (1H, d, J=Hz).

Example 2

To a mixture of sodium iodide (12.25 g), 5-methylpyrimidine (1.867 g), water (2.5 ml) and acetonitrile (7.5 ml) was added 7-[2-ethoxyimino-2-(5-amino-1,2,4-thiadiazol-3-yl)acetamido)-3-acetoacetoxymethyl-3-cephem-4-carboxylic acid (syn isomer) (5.0 g) at 60°C under stirring, which was continued for one hour at the same temperature. The reaction mixture was cooled to ambient temperature, diluted with a mixture of water (150 ml) and ethyl acetate (100 ml) and then adjusted to pH 2 with 1N hydrochloric acid. The aqueous layer was separated out, washed with ethyl acetate, evaporated to remove ethyl acetate and subjected to column chromatography on a non ionic adsorption resin Diaion HP—20 (150 ml). After the column was washed with water, the elution was carried out with 30% aqueous methanol. The eluants containing an object compounds were collected, concentrated to 40 ml under reduced pressure, passed through acidic alumina (10 g) and lyophilized to give 7-[2-ethoxyimino-2-(5-amino-1,2,4-thiadiazol-3-yl)acetamido]-3-(5-methyl-1-pyrimidiniomethyl)-3-cephem-4-carboxylate (syn isomer) (1.45 g), mp. 135 to 140°C (dec.).

IR (Nujol): 3250, 3150, 1770, 1660, 1610, 1520 $cm^{-1}$.

NMR ($D_2O$, δ): 1.32 (3H, t, J=7Hz), 2.58 (3H, s), 3.28 and 3.72 (2H, ABq, J=18Hz), 4.33 (2H, q, J=Hz), 5.30 (1H, d, J=5Hz), 5.37 and 5.60 (2H, ABq, J=14Hz), 5.90 (1H, d, J=Hz), 9.25 (2H, s), 9.60 (1H, s).

Example 3

The following compounds were obtained according to similar manners to those of Examples 1 and 2.

(1) 7-[2-Ethoxyimino-2-(5-amino-1,2,4-thiadiazol-3-yl)-acetamido]-3-(2-methyl-1-pyrazoliomethyl)-3-cephem-4-carboxylate (syn isomer), mp. 148 to 158°C (dec.).

IR (Nujol): 3400—3100, 1770, 1665, 1610, 1530 $cm^{-1}$.

NMR ($D_2O$, δ): 1.34 (3H, t, J=7Hz), 3.19, 3.57 (2H, ABq, J=18Hz), 4.11 (3H, s), 4.37 (2H, q, J=7Hz), 5.27 (1H, d, J=5Hz), 5.23 and 5.57 (2H, ABq, J=15Hz), 5.88 (1H, d, J=5Hz), 6.78 (1H, t, J=3Hz), 8.21 (2H, d, J=3Hz).

(2) 7-[2-Ethoxyimino-2-(5-amino-1,2,4-thiadiazol-3-yl)acetamido]-3-(1-pyridaziniomethyl)-3-cephem-4-carboxylate (syn isomer), mp. 155 to 160°C (dec.).

IR (Nujol): 3400—3100, 1770, 1660, 1610, 1530, 1350, 1040 $cm^{-1}$.

NMR ($D_2O$,δ): 1.23 (3H, t, J=7Hz), 3.03—3.92 (2H, m), 4.20 (2H, q, J=7Hz), 5.10 (1H, d, J=5Hz), 5.4—5.9 (2H, m), 5.77 (1H, d, J=5Hz), 7.67 (1H, m), 8.42 (1H, m), 9.20 (1H, m), 9.52 (1H, m).

(3) 7-[2-Ethoxyimino-2-(5-amino-1,2,4-thiadiazol-3-yl)acetamido]-3-(1-pyraziniomethyl)-3-cephem-4-carboxylate (syn isomer), mp. 165 to 170°C (dec.).

IR (Nujol): 3250, 3150, 1770, 1660, 1610, 1520 $cm^{-1}$.

NMR ($D_2O$, δ): 1.30 (3H, t, J=7Hz), 3.27 and 3.72 (2H, ABq, J=18Hz), 4.33 (2H, q, J=7Hz), 5.30 (1H, d, J=5Hz), 5.47 and 5.75 (2H, ABq, J=14Hz), 5.90 (1H, d, J=5Hz), 9.13 (2H, bs), 9.45 (2H, bs).

(4) 7-[2-Methoxyimino-2-(5-amino-1,2,4-thiadiazol-3-yl)acetamido]-3-(1-pyridaziniomethyl)-3-cephem-4-carboxylate (syn isomer), mp. 165 to 170°C (dec.).

IR (Nujol): 3400—3100, 1770, 1660—1580, 1530, 1040 $cm^{-1}$.

NMR ($D_2O$, δ): 3.47 and 3.83 (2H, ABq, J=18Hz), 4.05 (3H, s), 5.25 (1H, d, J=5Hz), 5.72 (2H, broad s), 5.87 (1H, d, J=Hz), 8.5—8.7 (2H, m), 9.4—9.6 (1H, m), 9.7—9.9 (1H, m).

(5) 7-[2-Ethoxyimino-2-(5-amino-1,2,4-thiadiazol-3-yl)acetamido]-3-(2-ethyl-1-pyrazoliomethyl)-3-cephem-4-carboxylate (syn isomer), mp. 150 to 155°C (dec.).

IR (Nujol): 3300, 3100, 1770, 1670, 1610, 1525 $cm^{-1}$.

NMR ($D_2O$, δ): 1.32 (3H, t, J=7Hz), 1.53 (3H, t, J=7Hz), 3.13 and 3.54 (2H, ABq, J=18Hz), 4.33 (4H, q, J=7Hz), 5.25 (1H, d, J=5Hz), 5.4 (2H, m), 5.85 (1H, d, J=5Hz), 6.7—6.9 (1H, m), 8.2—8.3 (2H, m).

(6) 7-[2-Allyloxyimino-2-(5-amino-1,2,4-thiadiazol-3-yl)acetamido]-3-(2-methyl-1-pyrazoliomethyl)-3-cephem-4-carboxylate (syn isomer), mp. 165 to 175°C (dec.).

IR (Nujol): 3200, 3100, 1770, 1670, 1610, 1520 $cm^{-1}$.

NMR ($D_2O$, δ): 3.20 and 3.60 (2H, ABq, J=18Hz), 4.13 (3H, s), 4.60—4.93 (2H, m), 5.30 (1H, d, J=5Hz), 5.16—5.63 (4H, m), 5.8—6.4 (2H, m), 6.80 (1H, t, J=3Hz), 8.20 (2H, d, J=3Hz).

(7) 7-[2-(2-Propynyloxyimino-2-(5-amino-1,2,4-thiadiazol-3-yl)acetamido]-3-(1-pyridaziniomethyl)-3-cephem-4-carboxylate (syn isomer), mp. 160 to 165°C (dec.).

IR (Nujol): 3250, 3200, 3100, 1770, 1660, 1620, 1520 $cm^{-1}$.

NMR ($D_2O$, δ): 3.03 (1H, t, J=2Hz), 3.48 and 3.83 (2H, ABq, J=18Hz), 4.93 (2H, d, J=2Hz), 5.27 (1H, d, J=5Hz), 5.73 (2H, broad s), 5.88 (1H, d, J=5Hz), 8.60 (2H, m), 9.47 (1H, m), 9.83 (1H, m).

(8) 7-[2-(2-Propynyloxyimino-2-(5-amino-1,2,4-thiadiazol-3-yl)acetamido]-3-(2-methyl-1-pyrazoliomethyl)-3-cephem-4-carboxylate (syn isomer), mp. 160 to 170°C (dec.).

IR (Nujol): 3250—3100, 1760, 1670, 1610, 1520 $cm^{-1}$.

NMR ($D_2O$, δ): 3.05 (1H, t, J=2Hz), 3.20 and 3.58 (2H, ABq, J=18Hz), 4.13 (3H, s), 4.97 (2H, d, J=2Hz), 5.30 (1H, d, J=5Hz), 5.27 and 5.57 (2H, ABq, J=14Hz), 5.92 (1H, d, J=Hz), 6.80 (1H, t, J=3Hz), 8.23 (2H, broad s).

(9) 7-[2-Methylthiomethoxyimino-2-(5-amino-1,2,4-thiadiazol-3-yl)acetamido]-3-(2-methyl-1-pyrazoliomethyl-3-cephem-4-carboxylate (syn isomer), mp. 145 to 150°C (dec.).

IR (Nujol): 3280, 3100, 1770, 1665, 1605, 1525 $cm^{-1}$.

NMR ($D_2O+CD_3OD$, δ): 2.23 (3H, s), 3.25 and 3.47 (2H, ABq, J=18Hz), 4.10 (3H, s), 5.25 (1H, d, J=5Hz), 5.35 (2H, s), 5.28 and 5.48 (2H, ABq, J=14Hz), 5.87 (1H, d, J=5Hz), 6.77 (1H, m), 8.20 (2H, m).

(10) 7-[2-Carboxymethoxyimino-2-(5-amino-1,2,4-thiadiazol-3-yl)acetamido]-3-(2-methyl-1-pyrazoliomethyl)-3-cephem-4-carboxylate (syn isomer), mp. 165 to 170°C (dec.).

IR (Nujol): 3400, 3290, 3160, 3110, 1770, 1670, 1615, 1525 $cm^{-1}$.

NMR ($D_2O+NaHCO_3$, δ): 3.27 and 3.47 (2H, ABq, J=18Hz), 4.10 (3H, s), 4.70 (2H, s), 5.27 (1H, d, J=5Hz), 5.32 and 5.45 (2H, ABq, J=14Hz), 5.92 (1H, d, J=5Hz), 6.78 (1H, m), 8.22 (2H, m).

(11) 7-[2-(2-Cyclopenten-1-yloxyimino-2-(5-amino-1,2,4-thiadiazol-3-yl)acetamido]-3-(2-methyl-1-pyrazoliomethyl)-3-cephem-4-carboxylate (syn isomer), mp 155 to 160°C (dec.).

IR (Nujol): 3250, 3100, 1770, 1660, 1605, 1525 $cm^{-1}$.

NMR ($D_2O+CD_3OD$, δ): 1.83—2.63 (4H, m), 3.20 and 3.43 (2H, ABq, J=18Hz), 4.12 (3H, s), 5.17 (1H, d, J=5Hz), 5.13—5.67 (3H, m), 5.80 (1H, d, J=5Hz), 5.73—6.33 (2H, m), 6.77 (1H, m), 8.20 (2H, m).

(12) 7-[2-Ethoxyimino-2-(5-amino-1,2,4-thiadiazol-3-yl)acetamido]-3-(2-allyl-1-pyrazoliomethyl)-3-cephem-4-carboxylate (syn isomer), mp. 130 to 140°C (dec.).

IR (Nujol): 3400—3100, 1770, 1660, 1610, 1530 $cm^{-1}$.

NMR ($D_2O$, δ): 1.32 (3H, t, J=7Hz), 3.13 and 3.46 (2H, ABq, J=18Hz), 4.31 (2H, q, J=7Hz), 5.0—5.3 (4H,

m), 5.20 (1H, d, J=5Hz), 5.29 and 5.39 (2H, ABq, J=14Hz), 5.82 (1H, d, J=5Hz), 5.8—6.2 (1H, m), 6.8 (1H, m), 8.2 (2H, m).

(13) 7-[2-Ethoxyimino-2-(5-amino-1,2,4-thiadiazol-3-yl)acetamido]-3-(3-chloro-1-pyridaziniomethyl)-3-cephem-4-carboxylate (syn isomer), mp. 145 to 150°C (dec.).
IR (Nujol): 3300—3100, 1775, 1670, 1610, 1530 $cm^{-1}$.

(14) 7-[2-Ethoxyimino-2-(5-amino-1,2,4-thiadiazol-3-yl)acetamido]-3-[3-methyl-1-(1,3,4-triazoliomethyl)]-3-cephem-4-carboxylate (syn isomer), mp. 153 to 157°C (dec.).
IR (Nujol): 3400—3100, 1770, 1660, 1610, 1530 $cm^{-1}$.
NMR (DMSO-$d_6$, δ): 1.22 (3H, t, J=7Hz), 3.3—3.7 (2H, m), 4.05 (3H, s), 4.13 (2H, q, J=7Hz), 4.7—5.3 (2H, m), 4.98 (1H, d, J=5Hz), 5.63 (1H, 2d, J=5 and 8Hz), 8.10 (2H, broad s), 9.30 (1H, s), 9.33 (1H, d, J=8Hz), 10.25 (1H, s).

(15) 7-[2-Ethoxyimino-2-(5-amino-1,2,4-thiadiazol-3-yl)acetamido]-3-[4-methyl-1-(1,2,4-triazoliomethyl)]-3-cephem-4-carboxylate (syn isomer), mp. 159 to 164°C (dec.).
IR (Nujol): 3400—3100, 1770, 1660, 1605, 1530 $cm^{-1}$.
NMR (DMSO-$d_6$, δ): 1.23 (3H, t, J=7Hz), 3.1—3.8 (2H, m), 3.91 (3H, s), 4.12 (2H, q, J=7Hz), 4.98 (1H, d, J=5Hz), 5.0—5.5 (2H, m), 5.63 (1H, 2d, J=5 and 8Hz), 8.13 (2H, broad s), 9.03 (1H, s), 9.31 (1H, d, J=8Hz), 10.25 (1H, s).

(16) 7-[2-Ethoxyimino-2-(5-amino-1,2,4-thiadiazol-3-yl)acetamido]-3-(3-hydroxy-1-pyridaziniomethyl)-3-cephem-4-carboxylate (syn isomer), mp. 159 to 163°C (dec.).
IR (Nujol): 3400—3100, 1775, 1670, 1610, 1520 $cm^{-1}$.
NMR (DMSO-$d_6$, δ): 1.27 (3H, t, J=7Hz), 3.56 (2H, broad s), 4.16 (2H, q, J=7Hz), 4.9—5.6 (3H, m), 5.83 (1H, 2d, J=5 and 8Hz), 6.91 (1H, d, J=9Hz), 7.6 (1H, m), 8.09 (2H, broad s), 8.44 (1H, d, J=4Hz), 9.54 (1H, d, J=8Hz).

(17) 7-[2-Ethoxyimino-2-(5-amino-1,2,4-thiadiazol-3-yl)acetamido]-3-(3-methyl-1-imidazoliomethyl)-3-cephem-4-carboxylate (syn isomer), mp. 156 to 165°C (dec.).
IR (Nujol): 3300, 3150, 1770, 1660, 1605, 1530 $cm^{-1}$.
NMR (DMSO-$d_6$, δ): 1.23 (3H, t, J=7Hz), 3.12 and 3.54 (2H, ABq, J=18Hz), 3.86 (3H, s), 4.15 (2H, q, J=7Hz), 5.02 (1H, d, J=5Hz), 5.02 and 5.18 (2H, ABq, J=14Hz), 5.66 (1H, 2d, J=5 and 9Hz), 7.65 (1H, s), 7.97 (1H, s), 8.25 (2H, broad s), 9.32 (1H, s), 9.45 (1H, d, J=9Hz).

## Example 4

A mixture of diphenylmethyl 7-[2-ethoxyimino-2-(5-amino-1,2,4-thiadiazol-3-yl)acetamido]-3-(3-methyl-1-imidazoliomethyl)-3-cephem-4-carboxylate trifluoroacetate (syn isomer) (3 g) and anisole (7.5 ml) in trifluoroacetic acid (20 ml) was stirred for 20 minutes under cooling in an ice bath. The mixture was poured into cold diisopropyl ether (120 ml) under stirring and resulting precipitates were collected by filtration. The powder was suspended in water (50 ml), adjusted to pH 4 to 5 with aqueous solution of sodium bicarbonate and an insoluble material was filtered off. The filtrate was subjected to column chromatography on a non ionic adsorption resin "Diaion HP—20"(Trademark: Prepared by Mitsubishi Chemical Industries) (100 ml). After the column was washed with water, the elution was carried out with 20% aqueous methanol. The eluates containing an object compound were collected, evaporated to remove methanol under reduced pressure and lyophilized to give 7-[2-ethoxyimino-2-(5-amino-1,2,4-thiadiazol-3-yl)acetamido]-3-(3-methyl-1-imidazoliomethyl)-3-cephem-4-carboxylate (syn isomer) (1.46 g), mp. 156 to 165°C (dec.).
IR (Nujol): 3300, 3150, 1770, 1660, 1605, 1530 $cm^{-1}$.
NMR (DMSO-$d_6$, δ): 1.23 (3H, t, J=7Hz), 3.12 and 3.54 (2H, ABq, J=18Hz), 3.86 (3H, s), 4.15 (2H, q, J=7Hz), 5.02 (1H, d, J=5Hz), 5.02 and 5.18 (2H, ABq, J=14Hz), 5.66 (1H, 2d, J=5 and 9Hz), 7.65 (1H, s), 7.97 (1H, s), 8.25 (2H, broad s), 9.32 (1H, s), 9.45 (1H, d, J=9Hz).

## Example 5

The following compounds were obtained according to a similar manner to that of Example 4.

(1) 7-[2-Ethoxyimino-2-(5-amino-1,2,4-thiadiazol-3-yl)acetamido]-3-[3-methyl-1-(1,2,3-triazolio)methyl]-3-cephem-4-carboxylate (syn isomer), mp. 141 to 145°C (dec.).
IR (Nujol): 3400—3100, 1770, 1660, 1610, 1530, 1035 $cm^{-1}$.

(2) 7-[2-Ethoxyimino-2-(5-amino-1,2,4-thiadiazol-3-yl)acetamido]-3-(5-methyl-1-pyrimidiniomethyl)-3-cephem-4-carboxylate (syn isomer), mp. 135 to 140°C (dec.).
IR (Nujol): 3250, 3150, 1770, 1660, 1610, 1520 $cm^{-1}$.

(3) 7-[2-Ethoxyimino-2-(5-amino-1,2,4-thiadiazol-3-yl)acetamido]-3-(2-methyl-1-pyrazoliomethyl)-3-cephem-4-carboxylate (syn isomer), mp. 148 to 158°C (dec.).
IR (Nujol): 3400—3100, 1770, 1665, 1610, 1530 $cm^{-1}$.

(4) 7-[2-Ethoxyimino-2-(5-amino-1,2,4-thiadiazol-3-yl)acetamido]-3-(1-pyridaziniomethyl)-3-cephem-4-carboxylate (syn isomer), mp. 155 to 160°C. (dec.).
IR (Nujol): 3400—3100, 1770, 1660, 1610, 1530, 1350, 1040 $cm^{-1}$.

(5) 7-[2-Ethoxyimino-2-(5-amino-1,2,4-thiadiazol-3-yl)acetamido]-3-(1-pyraziniomethyl)-3-cephem-4-carboxylate (syn isomer), mp. 165 to 170°C (dec.).
IR (Nujol): 3250, 3150, 1770, 1660, 1610, 1520 $cm^{-1}$.

(6) 7-[2-Methoxyimino-2-(5-amino-1,2,4-thiadiazol-3-yl)acetamido]-3-(1-pyridaziniomethyl)-3-cephem-4-carboxylate (syn isomer), mp. 165 to 170°C (dec.).
IR (Nujol): 3400—3100, 1770, 1660—1580, 1530, 1040 $cm^{-1}$.

(7) 7-[2-Ethoxyimino-2-(5-amino-1,2,4-thiadiazol-3-yl)acetamido]-3-(2-ethyl-1-pyrazoliomethyl)-3-cephem-4-carboxylate (syn isomer), mp. 150 to 155°C (dec.).
IR (Nujol): 3300, 3100, 1770, 1670, 1610, 1525 $cm^{-1}$.

(8) 7-[2-Allyloxyimino-2-(5-amino-1,2,4-thiadiazol-3-yl)acetamido]-3-(2-methyl-1-pyrazoliomethyl)-3-cephem-4-carboxylate (syn isomer), mp. 165 to 175°C (dec.).
IR (Nujol): 3200, 3100, 1770, 1670, 1610, 1520 $cm^{-1}$.

(9) 7-[2-(2-Propynyloxyimino-2-(5-amino-1,2,4-thiadiazol-3-yl)acetamido]-3-(1-pyridaziniomethyl)-3-cephem-4-carboxylate (syn isomer), mp. 160 to 165°C (dec.).
IR (Nujol): 3250, 3200, 3100, 1770, 1660, 1620, 1520 $cm^{-1}$.

(10) 7-[2-(2-Propynyloxyimino-2-(5-amino-1,2,4-thiadiazol-3-yl)acetamido]-3-(2-methyl-1-pyrazoliomethyl)-3-cephem-4-carboxylate (syn isomer), mp. 160 to 170°C (dec.).
IR (Nujol): 3250—3100, 1760, 1670, 1610, 1520 $cm^{-1}$.

(11) 7-[2-Methylthiomethoxyimino-2-(5-amino-1,2,4-thiadiazol-3-yl)acetamido]-3-(2-methyl-1-pyrazoliomethyl)-3-cephem-4-carboxylate (syn isomer), mp. 145 to 150°C (dec.).
IR (Nujol): 3280, 3100, 1770, 1665, 1605, 1525 $cm^{-1}$.

(12) 7-[2-Carboxymethoxyimino-2-(5-amino-1,2,4-thiadiazol-3-yl)acetamido]-3-(2-methyl-1-pyrazoliomethyl)-3-cephem-4-carboxylate (syn isomer), mp. 165 to 170°C (dec.).
IR (Nujol): 3400, 3290, 3160, 3110, 1770, 1670, 1615, 1525 $cm^{-1}$.

(13) 7-[2-(2-Cyclopenten-1-yloxyimino-2-(5-amino-1,2,4-thiadiazol-3-yl)acetamido]-3-(2-methyl-1-pyrazoliomethyl)-3-cephem-4-carboxylate (syn isomer), mp. 155 to 160°C (dec.).
IR (Nujol): 3250, 3100, 1770, 1660, 1605, 1525 $cm^{-1}$.

(14) 7-[2-Ethoxyimino-2-(5-amino-1,2,4-thiadiazol-3-yl)acetamido]-3-(2-allyl-1-pyrazoliomethyl)-3-cephem-4-carboxylate (syn isomer), mp. 130 to 140°C (dec.).
IR (Nujol): 3400—3100, 1770, 1660, 1610, 1530 $cm^{-1}$.

(15) 7-[2-Ethoxyimino-2-(5-amino-1,2,4-thiadiazol-3-yl)acetamido]-3-(3-chloro-1-pyridaziniomethyl)-3-cephem-4-carboxylate (syn isomer), mp. 145 to 150°C (dec.).
IR (Nujol): 3300—3100, 1775, 1670, 1610, 1530 $cm^{-1}$.

(16) 7-[2-Ethoxyimino-2-(5-amino-1,2,4-thiadiazol-3-yl)acetamido]-3-[3-methyl-1-(1,3,4-triazoliomethyl)]-3-cephem-4-carboxylate (syn isomer), mp. 153 to 157°C (dec.).
IR (Nujol): 3400—3100, 1770, 1660, 1610, 1530 $cm^{-1}$.

(17) 7-[2-Ethoxyimino-2-(5-amino-1,2,4-thiadiazol-3-yl)acetamido]-3-[4-methyl-1-(1,2,4-triazoliomethyl]-3-cephem-4-carboxylate (syn isomer), mp. 159 to 164°C (dec.).
IR (Nujol): 3400—3100, 1770, 1660, 1605, 1530 $cm^{-1}$.

(18) 7-[2-Ethoxyimino-2-(5-amino-1,2,4-thiadiazol-3-yl)acetamido]-3-(3-hydroxy-1-pyridaziniomethyl)-3-cephem-4-carboxylate (syn isomer), mp. 159 to 163°C (dec.).
IR (Nujol): 3400—3100, 1775, 1670, 1610, 1520 $cm^{-1}$.

Preparation 2

To a solution of phosphorus pentachloride (54.6 g) in methylene chloride (500 ml) was added 2-ethoxyimino-2-(5-amino-1,2,4-thiadiazol-3-yl)acetic acid (syn isomer) (54.0 g) under stirring and cooling at −20°C. The mixture was stirred for 30 minutes at −15 to 12°C and for 2 hours at −5°C. To the mixture containing precipitates of an object compound was added diisopropyl ether (500 ml) at −5°C and the mixture was stirred for 30 minutes at −5 to 10°C. The resulting precipitates were collected by filtration, washed with diisopropyl ether and dried to give 2-ethoxyimino-2-(5-amino-1,2,4-thiadiazol-3-yl)acetyl chloride hydrochloride (syn isomer) (60.17 g), mp. 125 to 127°C (dec.).

IR (Nujol): 1785, 1625, 1055 $cm^{-1}$.

Analysis for $C_6H_8O_2N_4SCl_2$

| | C | H | N | S | Cl |
|---|---|---|---|---|---|
| calc'd: | 26.57 | 2.95 | 20.66 | 11.81 | 26.20 |
| found: | 26.13 | 2.99 | 20.49 | 11.77 | 26.41 |

Preparation 3

To a suspension of diphenylmethyl 7-amino-3-chloromethyl-3-cephem-4-carboxylate hydrochloride (27 g) in methylene chloride (300 ml) was added, N,N-dimethylaniline (36.2 g) under cooling in an ice bath at 5°C. To the solution was added portionwise 2-ethoxyimino-2-(5-amino-1,2,4-thiadiazol-3-yl)acetyl chloride hydrochloride (syn isomer) (16.2 g) below 11°C and the mixture was stirred for 45 minutes at 5°C. The reaction mixture was diluted with a mixed solvent of methylene chloride (100 ml) and water (200 ml) and adjusted to pH 2 with 1N hydrochloric acid. The organic layer was separated out, washed with water, dried over magnesium sulfate and evaporated to dryness. The residue was triturated in diethyl ether to give diphenylmethyl 7-[2-ethoxyimino-2-(5-amino-1,2,4-thiadiazol-3-yl)acetamido]-3-chloromethyl-3-cephem-4-carboxylate (syn isomer) (32.4 g), mp. 120 to 125°C (dec.).

IR (Nujol): 3300, 3150, 1780, 1725, 1675, 1625, 1530, 1495 $cm^{-1}$.

NMR (DMSO-$d_6$, δ): 1.28 (3H, t, J=7Hz), 3.68, (2H, broad s), 4.23 (2H, q, J=7Hz), 4.47 (2H, s), 5.27 (1H, d, J=5Hz), 5.97 (1H, 2d, J=5 and 8Hz), 7.0 (1H, s), 7.2—7.7 (10H, m), 8.17 (2H, broad s), 9.62 (1H, d, J=8Hz).

Preparation 4

To a cold solution of diphenylmethyl 7-[2-ethoxyimino-2-(5-amino-1,2,4-thiadiazol-3-yl)acetamido]-3-chloromethyl-3-cephem-4-carboxylate (syn isomer) (10 g) in a mixd solution of methylene chloride (100 ml) and acetic acid (10 ml) were added 30% hydrogen peroxide (1.84 ml) and sodium tungstate (0.3 g).

The mixture was stirred for 45 minutes in an ice bath and poured into diethyl ether (300 ml). The precipitates were collected by filtration and washed with diethyl ether to give diphenylmethyl 7-[2-ethoxyimino-2-(5-amino-1,2,4-thiadiazol-3-yl)acetamido]-3-chloromethyl-3-cephem-4-carboxylate-1- oxide (syn isomer) (8.9 g), mp 150 to 155°C (dec.).

IR (Nujol): 3280, 3170, 1785, 1723, 1667, 1628, 1530 $^{-1}$.

NMR (DMSO-$d_6$, δ): 1.30 (3H, t, J=7Hz), 3.90 (2H, broad s), 4.23 (2H, q, J=7Hz), 4.58 (2H, broad s), 5.12 (1H, d, J=5Hz), 6.10 (1H, 2d, J=5 and 8Hz), 7.02 (1H, s), 7.20—7.73 (10H, m), 8.15 (2H, broad s), 9.00 (1H, d, J=8Hz).

Preparation 5

To a cold solution of diphenylmethyl 7-[2-ethoxyimino-2-(5-amino-1,2,4-thiadiazol-3-yl)acetamido]-3-chloromethyl-3-cephem-4-carboxylate-1-oxide (syn isomer) (9.85 g) in acetone (222 ml) was added sodium iodide (8.22 g) and the mixture was stirred for 2 hours under cooling in an ice bath. The solvent was evaporated under reduced pressure and the residue was dissolved in a mixture of methylene chloride (200 ml) and water (100 ml). The organic layer was separated out, washed with an aqueous solution of sodium thiosulfate and water successively, dried over magnesium sulfate and evaporated to dryness. The residue was triturated in diethyl ether to give diphenylmethyl 7-[2-ethoxy-imino-2-(5-amino-1,2,4-thiadiazol-3-yl)-acetamido]-3-iodomethyl-3-cephem-4-carboxylate-1-oxide (syn isomer) (10.39 g), mp. 159 to 163°C [dec.).

IR (Nujol): 3250, 3150, 1780, 1720, 1670, 1620, 1520 $cm^{-1}$.

NMR (DMSO-$d_6$, δ): 1.29 (3H, t, J=7Hz), 3.95 (2H, m), 4.25 (2H, q, J=7Hz), 4.50 (2H, m), 5.12 (1H, d, J=5Hz), 6.05 (1H, 2d, J=5 and 9Hz), 7.00 (1H, s), 7.4 (10H, m), 8.13 (2H, broad s), 8.96 (1H, d, J=9Hz).

Preparation 6

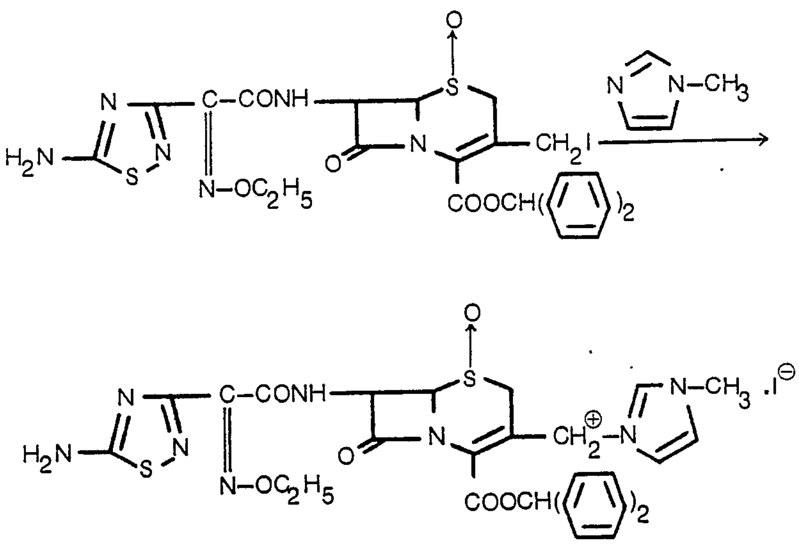

A mixture of diphenylmethyl 7-[2-ethoxyimino-2-(5-amino-1,2,4-thiadiazol-3-yl)acetamido]-3-iodomethyl-3-cephem-4-carboxylate-1-oxide (syn isomer) (4 g) and 1-methylimidazole (0.5 g) in tetrahydrofuran (7 ml) was stirred for 40 minutes at room temperature. The resulting precipitates were collected by filtration, washed with tetrahydrofuran and dried to give diphenylmethyl 7-[2-ethoxyimino-2-(5 - amino - 1,2,4 - thiadiazol - 3 - yl)acetamido] - 3 - (3 - methyl - 1 - imidazoliomethyl) - 3 - cephem - 4 - carboxylate - 1 - oxide iodide (syn isomer) (4.20 g), mp. 105 to 114°C (dec.).

IR (Nujol): 3250, 3150, 1790, 1720, 1670, 1610, 1520 $cm^{-1}$.

NMR (DMSO-$d_1$, δ): 1.21 (3H, t, J=7Hz), 3.77 (3H, s), 4.10 (2H, m), 4.15 (2H, q, J=7Hz), 5.1 (3H, m), 6.07 (2d, 1H, J=5 and 8Hz), 6.87 (1H, s), 7.33 (11H, m), 7.60 (1H, s), 8.00 (2H, broad s), 8.91 (1H, d, J=8Hz), 8.93 (1H, broad s).

Preparation 7

To a mixture of diphenylmethyl 7-[2-ethoxyimino-2-(5-amino-1,2,4-thiadiazol-3-yl)acetamido]-3-(3-methyl-1-imidazoliomethyl)-3-cephem-4-carboxylate-1-oxide iodide (syn isomer) (4 g) and N,N-dimethylaniline (1.21 g) in acetonitrile (40 ml) was dropped phosphorus trichloride (1.37 g) below 8°C under stirring and cooling in an ice bath. The mixture was stirred for 2.5 hours at the same temperature and diethyl ether (45 ml) was added thereto. The resulting precipitates were collected by filtration, washed with diethyl ether and dried to give diphenylmethyl 7-[2-ethoxyimino-2-(5-amino-1,2,4-thiadiazol-3-yl)acetamido]-3-(3-methyl-1-imidazoliomethyl)-3-cephem-4-carboxylate iodide (syn isomer) (4.18 g), mp. 115.5 to 120.5°C (dec.).

IR (Nujol): 1780, 1720, 1680, 1630, 1530 $cm^{-1}$.

NMR (DMSO-$d_6$, δ): 1.22 (3H, t, J=7Hz), 3.09 (3H, s), 3.86 (2H, m), 4.18 (2H, q, J=Hz), 5.18 (3H, m), 6.05 (1H, 2d, J=5 and 9Hz), 6.94 (1H, s), 7.4 (15H, m), 9.60 (1H, d, J=9Hz).

Preparation 8

A solution of diphenylmethyl 7-[2-ethoxyimino-2-(5-amino-1,2,4-thiadiazol-3-yl)acetamido]-3-(3-methyl-1-imidazoliomethyl)-3-cephem-4-carboxylate iodide (syn isomer) (3.9 g) in a mixed solvent (10 ml) of tetrahydrofuran and methanol (1:1) was subjected to a column packed with an ion-exchange resin (Amberlite IRA—400 (trademark: Prepared by Rohm and Haas Co.) trifluoroacetate-form] (40 ml). The elution was carried out with the same solvent (80 ml) and the eluate was evaporated to dryness. The residue was triturated in diethyl ether to give diphenylmethyl 7-[2-ethoxyimino-2-(5-amino-1,2,4-thiadiazol-3-yl)acetamido]-3-(3-methyl-1-imidazoliomethyl)-3-cephem-4-carboxylate trifluoroacetate (syn isomer) (2.74 g), mp. 110 to 119.5°C (dec.).

IR (Nujol): 3250, 3150, 1790, 1720, 1690, 1670, 1630, 1530, 1500 $cm^{-1}$.

Preparation 9

The following compound was obtained according to a similar manner to that of Preparation 1.

7-[2-Methylthiomethoxyimino-2-(5-amino-1,2,4-thiadiazol-3-yl)acetamido]cephalosporanic acid (syn isomer), mp;. 95.5 to 103.5°C (dec.).

IR (Nujol): 3400, 3300, 3190, 1770, 1720, 1670, 1620, 1525 $cm^{-1}$.

NMR (DMSO-$d_6$, δ): 2.00 (3H, s), 2.17 (3H, s), 3.50 (2H, broad s), 4.68 and 4.92 (2H, ABq, J=13Hz), 5.10 (1H, d, J=5Hz), 5.20 (2H, s), 5.77 (1H, 2d, J=5 and 8Hz), 8.07 (2H, broad s), 9.53 (1H, d, J=8Hz).

Example 6

The following compounds were obtained according to similar manners to those of aforesaid Examples.

(1) 7-[2-Ethoxyimino-2-(5-amino-1,2,4-thiadiazol-3-yl)acetamido]-3-[2-(2-hydroxyethyl)-1-pyrazoliomethyl]-3-cephem-4-carboxylate (syn isomer), mp 140 to 147°C (dec.).

IR (Nujol): 3400—3100, 1770, 1660, 1610, 1525, 1280, 1035 $cm^{-1}$.

NMR ($D_2O$, δ): 1.35 (3H, t, J=7Hz), 3.17 and 3.55 (2H, ABq, J=18Hz), 3.99 (2H, t, J=5Hz), 4.37 (2H, q, J=7Hz), 4.72 (2H, t, J=5Hz), 5.30 (1H, d, J=5Hz), 5.48 (2H, broad s), 5.92 (1H, d, J=5Hz), 6.89 (1H, m), 8.35 (2H, m).

(2) 7-[2-Ethoxyimino-2-(5-amino-1,2,4-thiadiazol-3-yl)acetamido]-3-(2-amino-1-pyrimidiniomethyl)-3-cephem-4-carboxylate (syn isomer), mp 195 to 200°C (dec.).

IR (Nujol): 3280, 3160, 1765, 1660, 1635, 1610, 1565, 1520, 1030 $cm^{-1}$.

NMR ($DMSO-d_6$, δ): 1.34 (3H, t, J=7Hz), 3.40 and 3.83 (2H, ABq, J=18Hz), 4.14 (2H, q, J=7Hz), 4.70 and 5.45 (2H, ABq, J=14Hz), 5.08 (1H, d, J=5Hz), 5.70 (1H, dd, J=5 and 8Hz), 7.0 (1H, m), 8.1 (2H, broad s), 8.5 (1H, m), 8.7 (1H, m), 9.46 (1H, d, J=8Hz).

**Claims**

1. A cephem compound of the formula:

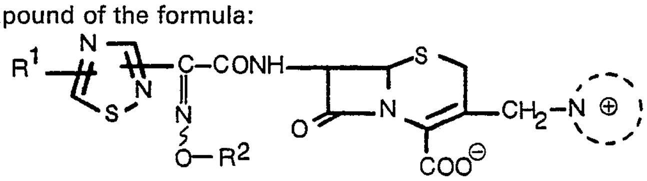

wherein

$R^1$ is amino or a protected amino group,

$R^2$ is $(C_{1-6})$alkyl, $(C_{2-6})$alkenyl or $(_{2-6})$alkynyl, each of which may be substituted with 1 to 3 substituents selected from $(C_{1-6})$alkylthio or carboxy, or cyclo$(C_{3-6})$alkenyl, and the group of the formula:

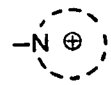

is triazolio, imidazolio, pyrimidinio, pyrazolio, pyridazinio or pyrazinio, each of which may be substituted with 1 to 3 substituents selected from $(C_{1-6})$alkyl, amino, $(C_{2-6})$alkenyl, halogen, hydroxy or hydroxy$(C_{1-6})$alkyl, with proviso that when $R^2$ is methyl or 2-carboxyprop-2-yl

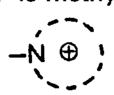

is not $(C_{1-6})$alkyl-triazolio or $(C_{1-6})$alkyl-pyrazolio; and a pharmaceutically acceptable salt thereof.

2. Syn isomer of a compound of claim 1, wherein

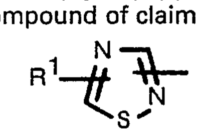 group is 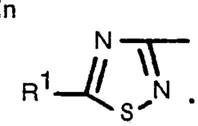 .

3. A compound of claim 2, wherein $R_1$ is amino, and $R_2$ and

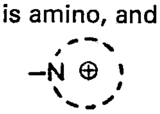

are as defined in claim 1.

4. A compound of claim 3, which is selected from the group consisting of:

7-[2-ethoxyimino-2-(5-amino-1,2,4-thiadiazol-3-yl)acetamido]-3-[3-methyl-1-(1,2,3-triazolio)methyl]-3-cephem-4-carboxylate (syn isomer),

7-[2-ethoxyimino-2-(5-amino-1,2,4-thiadiazol-3-yl)acetamido]-3-[3-methyl-1-(1,3,4-triazolio)methyl]-3-cephem-4-carboxylate (syn isomer),

7-[2-ethoxyimino-2-(5-amino-1,2,4-thiadiazol-3-yl)acetamido]-3-[4-methyl-1-(1,2,4-triazolio)methyl]-3-cephem-4-carboxylate (syn isomer).

5. A compound of claim 3, which is selected from the group consisting of:

7-[2-ethoxyimino-2-(5-amino-1,2,4-thiadiazol-3-yl)acetamido]-3-(2-methyl-1-pyrazoliomethyl)-3-cephem-4-carboxylate (syn isomer),

7-[2-ethoxyimino-2-(5-amino-1,2,4-thiadiazol-3-yl)acetamido]-3-(2-ethyl-1-pyrazoliomethyl)-3-cephem-4-carboxylate (syn isomer),

7-[2-allyloxyimino-2-(5-amino-1,2,4-thiadiazol-3-yl)acetamido]-3-(2-methyl-1-pyrazoliomethyl)-3-cephem-4-carboxylate (syn isomer),

7-[2-(2-propynyloxyimino)-2-(5-amino-1,2,4-thiadiazol-3-yl)acetamido]-3-(2-methyl-1-pyrazoliomethyl)-3-cephem-4-carboxylate (syn isomer),

7-[2-methylthiomethoxyimino-2-(5-amino-1,2,4-thiadiazol-3-yl)acetamido]-3-(2-methyl-1-pyrazoliomethyl)-3-cephem-4-carboxylate (syn isomer),

7-[2-carboxymethoxyimino-2-(5-amino-1,2,4-thiadiazol-3-yl)acetamido]-3-(2-methyl-1-pyrazoliomethyl)-3-cephem-4-carboxylate (syn isomer),

7-[2-(2-cyclopenten-1-yloxyimino)-2-(5-amino-1,2,4-thiadiazol-3-yl)acetamido]-3-(2-methyl-1-pyrazoliomethyl)-3-cephem-4-carboxylate (syn isomer),

7-[2-ethoxyimino-2-(5-amino-1,2,4-thiadiazol-3-yl)acetamido]-3-(2-allyl-1-pyrazoliomethyl)-3-cephem-4-carboxylate (syn isomer) and,

7-[2-ethoxyimino-2-(5-amino-1,2,4-thiadiazol-3-yl)acetamido]-3-[2-(2-hydroxyethyl)-1-pyrazoliomethyl]-3-cephem-4-carboxylate (syn isomer).

6. A compound of claim 3, which is selected from the group consisting of:

7-[2-ethoxyimino-2-(5-amino-1,2,4-thiadiazol-3-yl)acetamido]-2-(1-pyridaziniomethyl)-3-cephem-4-carboxylate (syn isomer),

7-[2-methoxyimino-2-(5-amino-1,2,4-thiadiazol-3-yl)acetamido]-3-(1-pyridaziniomethyl)-3-cephem-4-carboxylate (syn isomer),

7-[2-propynyloxyimino)-2-(5-amino-1,2,4-thiadiazol-3-yl)acetamido]-3-(1-pyridaziniomethyl)-3-cephem-4-carboxylate (syn isomer),

7-[2-ethoxyimino-2-(5-amino-1,2,4-thiadiazol-3-yl)acetamido]-3-(3-chloro-1-pyridaziniomethyl)-3-cephem-4-carboxylate (syn isomer), and

7-[2-ethoxyimino-2-(5-amino-1,2,4-thiadiazol-3-yl)acetamido]-3-(3-hydroxy-1-pyridaziniomethyl)-3-cephem-4-carboxylate (syn isomer).

7. A process for preparing a cephem compound of the formula:

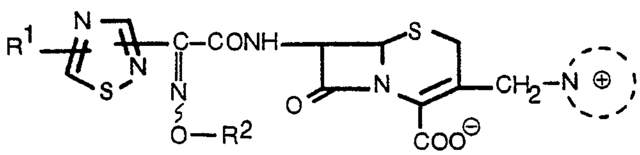

wherein

$R^1$ is amino or a protected amino group,

$R^2$ is $(C_{1-6})$alkyl, $(C_{2-6})$alkenyl or $(C_{2-6})$alkynyl, each of which may be substituted with 1 to 3 substituents selected from $(C_{1-6})$alkylthio or carboxy, or cyclo$(C_{3-6})$alkenyl, and the group of the formula:

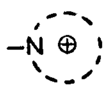

is triazolio, imidazolio, pyrimidinio, pyrazolio, pyridazinio or pyrazinio, each of which may be substituted with 1 to 3 substituents selected from $(C_{1-6})$alkyl, amino, $(C_{2-6})$alkenyl, halogen, hydroxy or hydroxy-$(C_{1-6})$alkyl, with proviso that when $R^2$ is methyl or 2-carboxyprop-2-yl

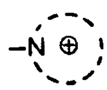

is not $(C_{1-6})$alkyl-triazolio or $(C_{1-6})$alkyl-pyrazolio; and a pharmaceutically acceptable salt thereof, which comprises

(a) reacting a compound of the formula:

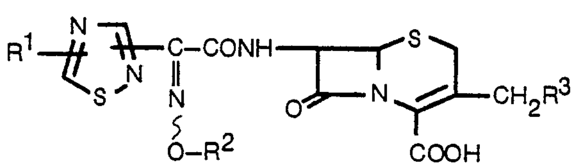

wherein

$R^1$ and $R^2$ are each as defined above and

$R^3$ is a group which can be replaced by a group of the formula:

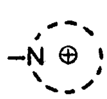

(wherein a group of the formula:

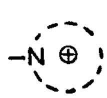

is as defined above; or a salt thereof, with a corresponding compound of the formula:

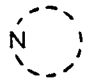

or

(b) subjecting a compound of the formula:

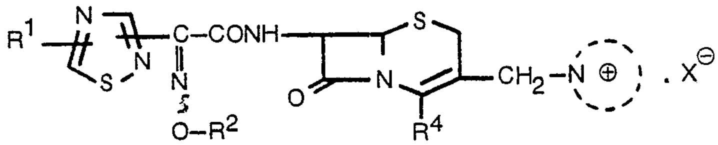

wherein

$R^1$, $R^2$ and the group of the formula:

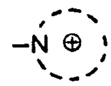

are each as defined above,

$R^4$ is a protected carboxy group and X is an acid residue, to elimination reaction of carboxy-protective group.

8. A compound of the formula:

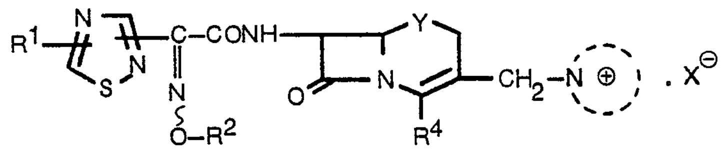

wherein

$R^1$ is as defined in claim 1,

$R^4$ is a protected carboxy group,

X is an acid residue,

Y is —S— or

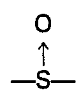

and

$R^2$ and

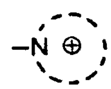

are as defined in claim 1, and a salt thereof.

9. A process for preparing a compound of the formula:

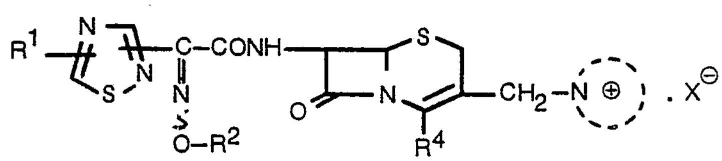

wherein

$R^1$ is amino or a protected amino group,

$R^4$ is a protected carboxy group,

X is an acid residue,

$R^2$ is $(C_{1-6})$alkyl, $(C_{2-6})$alkenyl or $(C_{2-6})$alkynyl, each of which may be substituted wth 1 to 3 substituents selected from $(C_{1-6})$alkylthio or carboxy, or cyclo$(C_{3-6})$alkenyl, and the group of the formula:

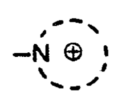

is triazolio, imidazolio, pyrimidinio, pyrazolio, pyridazinio, or pyrazinio, each of which may be substituted with 1 to 3 substituents selected from $(C_{1-6})$alkyl, amino, $(C_{2-6})$alkenyl, halogen, hydroxy or hydroxy-$(C_{1-6})$alkyl, with proviso that when $R^2$ is methyl or 2-carboxyprop-2-yl

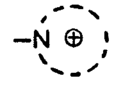

is not $(C_{1-6})$alkyl-triazolio or $(C_{1-6})$alkyl-pyrazolio; which comprises reducing a compound of the formula:

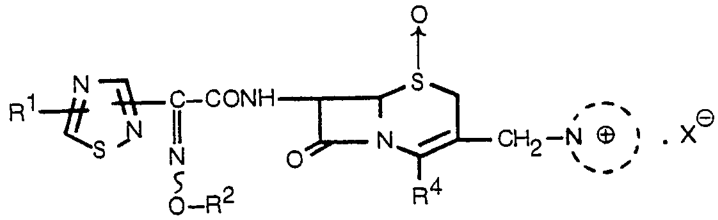

wherein $R^1$, $R^2$, $R^4$, X and the group of the formula:

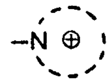

are each as defined above.

10. A pharmaceutical antibacterial composition comprising an unprotected compound of claim 1 in association with a pharmaceutically acceptable, substantially non-toxic carrier or excipient.

11. An unprotected compound as defined in any of claims 1 to 6 for use as a pharmaceutical.

12. An unprotected compound as defined in any of claims 1 to 6 for use in the treatment of infectious diseases.

**Patentansprüche**

1. Cephem-Verbindung der Formel

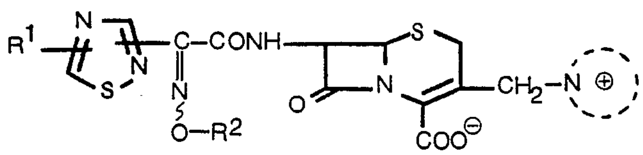

worin darstellen

$R^1$ Amino oder eine geschützte Aminogruppe,

$R^2$ $(C_{1-6})$-Alkyl, $(C_{2-6})$-Alkenyl oder $(C_{1-6})$-Alkinyl, von denen jedes substituiert sein kann durch 1 bis 3 Substituenten, ausgewählt aus $(C_{1-6})$-Alkylthio oder Carboxy oder Cyclo$(C_{3-6})$-alkenyl und die Gruppe der Formel

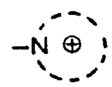

darstellt Triazolio, Imidazolio, Pyrimidinio, Pyrazolio, Pyridazinio oder Pyrazinio, von denen jedes substituiert sein kann durch 1 bis 3 Substituenten, ausgewählt aus $(C_{1-6})$-Alkyl, Amino, $(C_{2-6})$-Alkenyl, Halogen, Hydroxy oder Hydroxy$(C_{1-6})$-alkyl, mit der Maßgabe, daß dann, wenn $R^2$ für Methyl oder 2-Carboxyprop-2-yl steht,

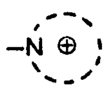

nicht $(C_{1-6})$-Alkyl-triazolio oder $(C_{-6})$-Alkyl-pyrazolio bedeutet;
sowie ein pharmazeutisch verträgliches Salz davon.

2. Syn-Isomeres einer Verbindung nach Anspruch 1, worin die

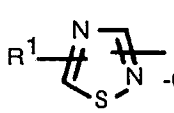 -Gruppe darstellt 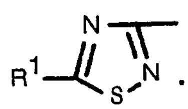 .

3. Verbindung nach Anspruch 2, worin $R^1$ für Amino steht und $R^2$ und

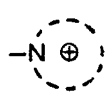

wie in Anspruch 1 definiert sind.

4. Verbindung nach Anspruch 3, die ausgewählt wird aus der Gruppe, die besteht aus:

7-[2-Ethoxyimino-2-(5-amino-1,2,4-thiadiazol-3-yl)acetamido]-3-[3-methyl-1-(1,2,3-triazolio)methyl]-3-cephem-4-carboxylat (syn-Isomeres),

7-[2-Ethoxyimino-2-(5-amino-1,2,4-thiadiazol-3-yl)acetamido]-3-[3-methyl-1-(1,3,4-triazolio)methyl]-3-cephem-4-carboxylat (syn-Isomeres) und

7-[2-Ethoxyimino-2-(5-amino-1,2,4-thiadiazol-3-yl)acetamido]-3-[4-methyl-1-(1,2,4-triazolio)methyl]-3-cephem-4-carboxylat (syn-Isomeres).

5. Verbindung nach Anspruch 3, die ausgewählt wird aus der Gruppe, die besteht aus:

7-[2-Ethoxyimino-2-(5-amino-1,2,4-thiadiazol-3-yl)acetamido]-3-(2-methyl-1-pyrazoliomethyl)-3-cephem-4-carboxylat (syn-Isomeres),

7-[2-Ethoxyimino-2-(5-amino-1,2,4-thiadiazol-3-yl)acetamido]-3-(2-ethyl-1-pyrazoliomethyl)-3-cephem-4-carboxylat (syn-Isomeres),

7-[2-Allyloxyimino-2-(5-amino-1,2,4-thiadiazol-3-yl)acetamido]-3-(2-methyl-1-pyrazoliomethyl)-3-cephem-4-carboxylat (syn-Isomeres),

7-[2-(2-Propinyloxyimino)-2-(5-amino-1,2,4-thiadiazol-3-yl)acetamido]-3-(2-methyl-1-pyrazoliomethyl)-3-cephem-4-carboxylat (syn-Isomeres),

7-[2-Methylthiomethoxyimino-2-(5-amino-1,2,4-thiadiazol-3-yl)acetamido]-3-(2-methyl-1-pyrazoliomethyl)-3-cephem-4-carboxylat (syn-Isomeres),

7-[2-Carboxymethoxyimino-2-(5-amino-1,2,4-thiadiazol-3-yl)acetamido]-3-(2-methyl-1-pyrazoliomethyl)-3-cephem-4-carboxylat (syn-Isomeres),

7-[2-(2-Cyclopenten-1-yloxyimino)-2-(5-amino-1,2,4-thiadiazol-3-yl)acetamido]-3-(2-methyl-1-pyrazoliomethyl)-3-cephem-4-carboxylat (syn-Isomeres),

7-[2-Ethoxyimino-2-(5-amino-1,2,4-thiadiazol-3-yl)acetamido]-3-(2-allyl-1-pyrazoliomethyl)-3-cephem-4-carboxylat (syn-Isomeres) und

7-[2-Ethoxyimino-2-(5-amino-1,2,4-thiadiazol-3-yl)acetamido]-3-[2-(2-hydroxyethyl)-1-pyrazoliomethyl]-3-cephem-4-carboxylat (syn-Isomeres).

6. Verbindung nach Anspruch 3, die ausgewählt wird aus der Gruppe, die besteht aus:

7-[2-Ethoxyimino-2-(5-amino-1,2,4-thiadiazol-3-yl)acetamido]-2-(1-pyridaziniomethyl)-3-cephem-4-carboxylat (syn-Isomeres),

7-[2-Methoxyimino-2-(5-amino-1,2,4-thiadiazol-3-yl)acetamido]-3-(1-pyridaziniomethyl)-3-cephem-4-carboxylat (syn-Isomeres),

7-[2-(2-Propinyloxyimino)-2-(5-amino-1,2,4-thiadiazol-3-yl)acetamido]-3-(1-pyridaziniomethyl)-3-cephem-4-carboxylat (syn-Isomeres),

7-[2-Ethoxyimino-2-(5-amino-1,2,4-thiadiazol-3-yl)acetamido]-3-(3-chloro-1-pyridaziniomethyl)-3-cephem-4-carboxylat (syn-Isomeres) und

7-[2-Ethoxyimino-2-(5-amino-1,2,4-thiadiazol-3-yl)acetamido]-3-(3-hydroxy-1-pyridaziniomethyl)-3-cephem-4-carboxylat (syn-Isomeres).

7. Verfahren zur Herstellung einer Cephem-Verbindung der Formel

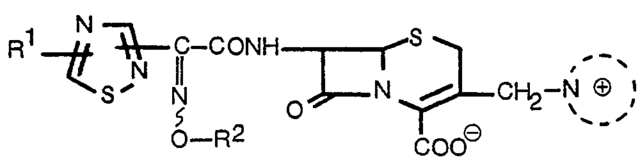

worin bedeuten

$R^1$ Amino oder eine geschützte Aminogruppe,

$R^2$ $(C_{1-6})$-Alkyl, $(C_{2-6})$-Alkenyl oder $(C_{2-6})$-Alkinyl, von denen jedes substituiert sein kann durch 1 bis 3 Substituenten, ausgewählt aus $(C_{1-6})$-Alkylthio oder Carboxy oder Cyclo$(C_{3-6})$-alkenyl und die Gruppe der

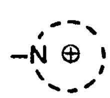

steht für Triazolio, Imidazolio, Pyrimidinio, Pyrazolio, Pyridazinio oder Pyrazinio, von denen jedes substituiert sein kann durch 1 bis 3 Substituenten, ausgewählt aus $(C_{1-6})$-Alkyl, Amino, $(C_{2-6})$-Alkenyl, Halogen, Hydroxy oder Hydroxy$(C_{1-6})$-alkyl, mit der Maßgabe, daß dann, wenn $R^2$ für Methyl oder 2-Carboxyprop-2-yl steht,

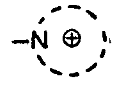

nicht $(C_{1-6})$-Alkyl-triazolio oder $(C_{1-6})$-Alkyl-pyrazolio bedeutet; und eines pharmazeutisch verträglichen Salzes davon, das umfaßt

a) die Umsetzung einer Verbindung der Formel

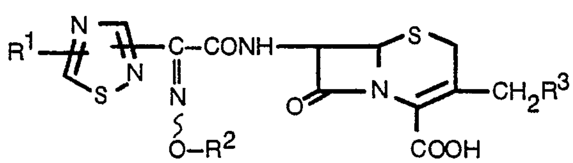

worin $R^1$ und $R^2$ jeweils wie oben definiert sind und $R_3$ eine Gruppe darstellt, die ersetzt sein kann durch eine Gruppe der Formel

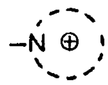

(worin eine Gruppe der Formel

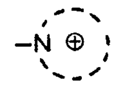

wie oben definiert ist)
oder eines Salzes davon mit einer entsprechenden Verbindung der Formel

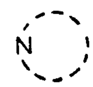

oder

b) die Durchführung einer Eliminierungsreaktion zur Eliminierung der Carboxyschutzgruppe mit einer Verbindung der Formel

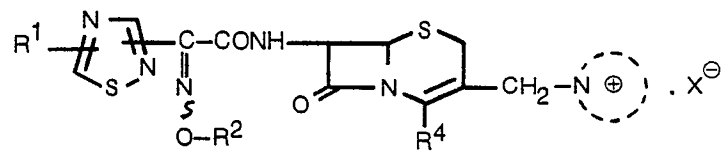

worin $R^1$, $R^2$ und die Gruppe der Formel

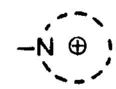

jeweils wie oben definiert sind, $R^4$ eine geschützte Carboxygruppe bedeutet und X für einen Säurerest steht.

8. Verbindung der Formel

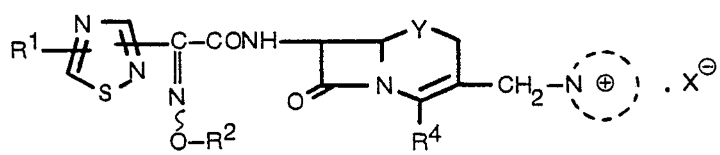

worin $R^1$ wie in Anspruch 1 definiert ist, $R^4$ eine geschützte Carboxygruppe bedeutet, X einen Säurerest bedeutet, Y —S— oder

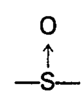

bedeutet und $R^2$ und

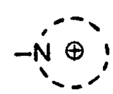

wie in Anspruch 1 definiert sind, und ein Salz davon.

9. Verfahren zur Herstellung einer Verbindung der Formel

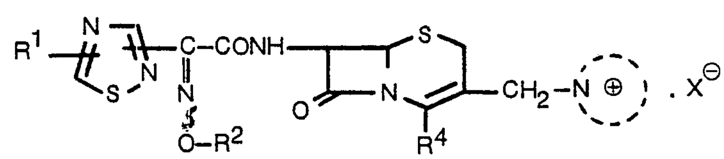

worin bedeuten:

$R^1$ Amino oder eine geschützte Aminogruppe,

$R^4$ eine geschützte Carboxygruppe,

X einen Säurerest,

$R^2$ $(C_{1-6})$-Alkyl, $(C_{2-6})$-Alkenyl oder $(C_{2-6})$-Alkinyl, von denen jedes substituiert sein kann durch 1 bis 3 Substituenten, ausgewählt aus $(C_{1-6})$-Alkylthio oder Carboxy oder Cyclo $(C_{3-6})$-alkenyl und die Gruppe der Formel

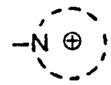

steht für Triazolio, Imidazolio, Pyrimidinio, Pyrazolio, Pyridazinio oder Pyrazinio, von denen jedes substituiert sein kann durch 1 bis 3 Substituenten, ausgewählt aus $(C_{1-6})$-Alkyl, Amino, $(C_{2-6})$-Alkenyl, Halogen, Hydroxy oder Hydroxy$(C_{1-6})$-alkyl, mit der Maßgabe, daß dann, wenn $R^2$ für Methyl oder 2-Carboxyprop-2-yl steht,

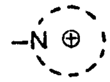

nicht $(C_{1-6})$-Alkyl-triazolio oder $(C_{1-6})$-Alkyl-pyrazolio bedeutet, das umfaßt die Reduktion einer Verbindung der Formel

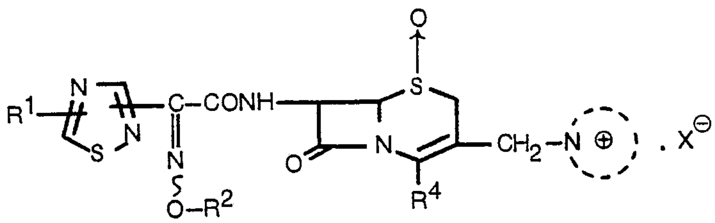

worin $R^1$, $R^2$, $R^4$, X und die Gruppe der Formel

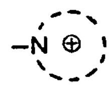

jeweils wie oben definiert sind.

10. Pharmazeutische antibakterielle Zusammensetzung, die eine ungeschützte Verbindung des Anspruchs 1 in Assoziation mit einem pharmazeutisch verträglichen, im wesentlichen nicht-toxischen Träger oder Exzipienten enthält.

11. Ungeschützte Verbindung nach einem der Ansprüche 1 bis 6 für die Verwendung als Pharmaceuticum.

12. Ungeschützte Verbindung nach einem der Ansprüche 1 bis 6 für die Verwendung zur Behandlung von Infektionserkrankungen.

**Revendications**

1. Céphème répondant à la formule:

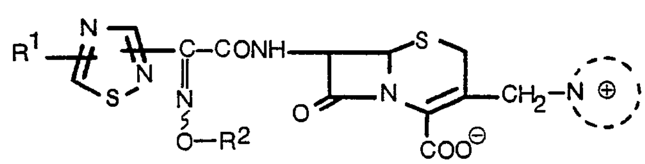

dans laquelle

$R^1$ est un groupe amino ou amino protégé,

$R^2$ est un groupe alkyle en $C_1$ à $C_6$, alcényle en $C_2$ à $C_6$ ou alcynyle en $C_2$ à $C_6$, chacun pouvant être substitué par 1 à 3 substituants choisis parmi des groupes alkylthio en $C_1$ à $_6$ ou carboxy ou cyclo(alcényle en $C_3$ à $C_6$) et le groupe répondant à la formule:

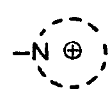

est un groupe triazolio, imidazolio, pyrimidinio, pyrazolio, pyridazinio ou pyrazinio, chacun pouvant être substitué par 1 à 3 substituants choisis parmi des groupes alkyle en $C_1$ -$C_6$, amino, alcényle en $C_2$ à $C_6$, halogène, hydroxy ou hydroxy(alkyle en $C_1$ à $C_6$)
sous réserve que lorsque $R^2$ est un groupe méthyle ou 2-carboxyprop-2-yl

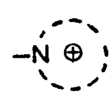

n'est pas un groupe (alkyle en $C_1$ à $C_6$)-triazolio ou (alkyle en $C_1$ à $C_6$)-pyrazolio;
et ses sels pharmaceutiquement acceptables.

2. Isomère syn d'un composé de la revendication 1 dans lequel

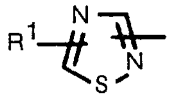 est un groupe 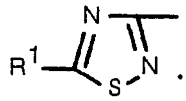.

3. Composé selon la revendication 2, dans lequel $R^1$ est un groupe amino, et $R^2$ et

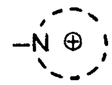

sont tels que définis dans la revendication 1.

4. Composé selon la revendication 3, qui est choisi dans le groupe constitué:

du 7-[2-éthoxyimino-2-(5-amino-1,2,4-thiadiazol-3-yl)acétamido]-3-[3-méthyl-1-(1,2,3-triazolio)méthyl]-3-céphème-4-carboxylate (isomère syn),

du 7-[2-éthoxyimino-2-(5-amino-1,2,4-thiadiazol-3-yl)acétamido]-3-[3-méthyl-1-(1,3,4-triazolio)méthyl]-3-céphème-4-carboxylate (isomère syn), et

du 7-[2-éthoxyimino-2-(5-amino-1,2,4-thiadiazol-3-yl)acétamido]-3-[4-méthyl-1-(1,2,4-triazolio)méthyl]-3-céphème-4-carboxylate (isomère syn).

5. Composé selon la revendication 3, qui est choisi dans le groupe constitué:

du 7-[2-éthoxyimino-2-(5-amino-1,2,4-thiadiazol-3-yl)acétamido]-3-(2-méthyl-1-pyrazoliométhyl)-3-céphème-4-carboxylate (isomère syn),

du 7-[2-éthoxyimino-2-(5-amino-1,2,4-thiadiazol-3-yl)acétamido]-3-(2-éthyl-1-pyrazoliométhyl)-3-céphème-4-carboxylate (isomère syn),

du 7-[2-allyloxyimino-2-(5-amino-1,2,4-thiadiazol-3-yl)acétamido]-3-(2-méthyl-1-pyrazoliométhyl)-3-céphème-4-carboxylate (isomère syn),

du 7-[2-(2-propynyloxyimino)-2-(5-amino-1,2,4-thiadiazol-3-yl)acétamido]-3-(2-méthyl-1-pyrazoliométhyl)-3-céphème-4-carboxylate (isomère syn),

du 7-[2-méthylthiométhoxyimino-2-(5-amino-1,2,4-thiadiazol-3-yl)acétamido]-3-(2-méthyl-1-pyrazoliométhyl)-3-céphème-4-carboxylate (isomère syn),

du 7-[2-carboxyméthoxyimino-2-(5-amino-1,2,4-thiadiazol-3-yl)acétamido]-3-(2-méthyl-1-pyrazoliométhyl)-3-céphème-4-carboxylate (isomère syn),

du 7-[2-(2-cyclopentène-1-yloxyimino)-2-(5-amino-1,2,4-thiadiazol-3-yl)acétamido]-3-(2-méthyl-1-pyrazoliométhyl)-3-céphème-4-carboxylate (isomère syn),

du 7-[2-éthoxyimino-2-(5-amino-1,2,4-thiadiazol-3-yl)acétamido]-3-(2-allyl-1-pyrazoliométhyl)-3-céphème-4-carboxylate (isomère syn) et,

du 7-[2-éthoxyimino-2-(5-amino-1,2,4-thiadiazol-3-yl)acétamido]-3-[2-(2-hydroxyéthyl)-1-pyrazoliométhyl]-3-céphème-4-carboxylate (isomère syn).

6. Composé selon la revendication 3, qui est choisi dans le groupe constitué:

du 7-[2-éthoxyimino-2-(5-amino-1,2,4-thiadiazol-3-yl)acétamido]-2-(1-pyridaziniométhyl)-3-céphème-4-carboxylate (isomère syn),

du 7-[2-méthoxyimino-2-(5-amino-1,2,4-thiadiazol-3-yl)acétamido]-3-(1-pyridaziniométhyl)-3-céphème-4-carboxylate (isomère syn),

du 7-[2-(2-propynyloxyimino)-2-(5-amino-1,2,4-thiadiazol-3-yl)acétamido]-3-(1-pyridaziniométhyl)-3-céphème-4-carboxylate (isomère syn),

du 7-[2-éthoxyimino-2-(5-amino-1,2,4-thiadiazol-3-yl)acétamido]-3-(3-chloro-1-pyridaziniométhyl)-3-céphème-4-carboxylate (isomère syn), et

du 7-[2-éthoxyimino-2-(5-amino-1,2,4-thiadiazol-3-yl)acétamido]-3-(3-hydroxy-1-pyridaziniométhyl)-3-céphème-4-carboxylate (isomère syn).

7. Procédé de préparation d'un céphème répondant à la formule:

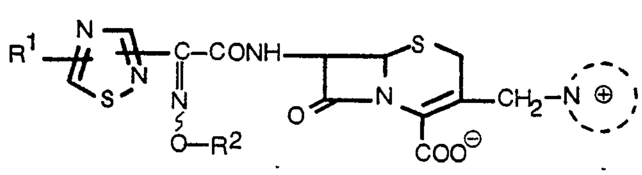

dans laquelle

$R^1$ est un groupe amino ou amino protégé,

$R^2$ est un groupe alkyle en $C_1$ à $C_6$, alcényle en $C_2$ à $C_6$, ou alcynyle en $C_2$ à $C_6$, chacun d'eux pouvant être substitué par 1 à 3 substituants choisis parmi les groupes alkylthio en $C_1$ à $C_6$ ou carboxy, ou cyclo(alcényle en $C_3$ à $C_6$), et le groupe répondant à la formule:

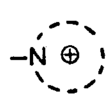

est un groupe triazolio, imidazolio, pyrimidinio, pyrazolio, pyridazinio ou pyrazinio, chacun d'eux pouvant être substitué par 1 à 3 substituants choisis parmi les groupes alkyle en $C_1$ à $C_6$, amino, alcényle en $C_2$ à $C_6$, halogène, hydroxy ou hydroxy (alkyl en $C_1$ à $C_6$),

sous réserve que lorsque $R^2$ est un groupe méthyle ou 2-carboxyprop-2-yle,

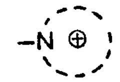

n'est pas un groupe (alkyle en $C_{1-6}$)-triazolio ou (alkyle en $C_{1-6}$)-pyrazolio;

et ses sels pharmaceutiquement acceptables, qui comprend:

(a) la réaction d'un composé répondant à la formule:

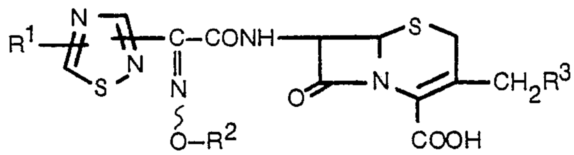

dans laquelle $R_1$ et $R_2$ sont chacun tels que définis ci-dessus, et

$R_3$ est un groupe qui peut être remplacé par un groupe répondant à la formule:

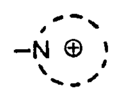

(dans laquelle le groupe répondant à la formule:

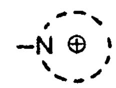

est tel que défini ci-dessus);

ou un de ses sels, avec un composé correspondant répondant à la formule

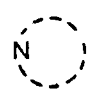

ou

(b) le fait de soumettre un composé répondant à la formule:

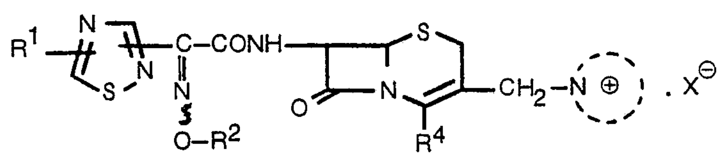

dans laquelle $R^1$, $R^2$ et le groupe répondant à la formule:

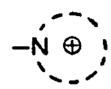

sont chacun tels que définis ci-déssus, $R^4$ est un groupe carboxy protégé et X est un radical acide, à une réaction d'élimination du groupe protecteur du groupe carboxy.

8. Composé répondant à la formule:

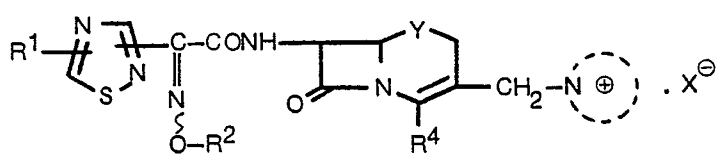

dans laquelle

$R^1$ est tel que défini dans la revendication 1,

$R^4$ est un groupe carboxy protégé,

X est un radical acide,

Y est —S— ou

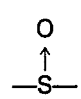

et

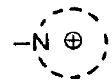

sont tels que définis dans la revendication 1, et un de ses sels.

9. Procédé de préparation d'un composé répondant à la formule:

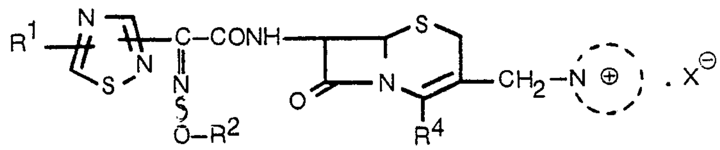

dans laquelle

$R^1$ est un groupe amino ou amino protégé,

$R^4$ est un groupe carboxy protégé,

X est un radical acide,

$R^2$ est un groupe alkyle en $C_1$ à $C_6$, alcényle en $C_2$ à $C_6$ ou alcynyle en $C_2$ à $C_6$, chacun d'eux pouvant être substitué par 1 à 3 substituants choisis parmi un groupe alkylthio en $C_1$ à $C_6$ ou carboxy, ou un groupe cycloalcényle en $C_3$ à $C_6$, et le groupe répondant à la formule:

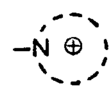

est un groupe triazolio, imidazolio, pyrimidinio, pyrazolio, pyridazinio, ou pyrazinio,

chacun d'eux pouvant être substitué par 1 à 3 substituants choisis parmi les groupes alkyle en $C_1$ à $C_6$, amino, alcényle en $C_2$ à $C_6$, halogène, hydroxy ou hydroxy(alkyle en $C_1$ à $C_6$),

sous réserve que lorsque $R_2$ est un groupe méthyle ou 2-carboxyprop-2-yle,

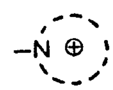

n'est pas un groupe (alkyle en $C_1$ à $C_6$)-triazolio, ou (alkyle en $C_1$ à $C_6$)-pyrazolio;

qui comprend la réduction d'un composé répondant à la formule:

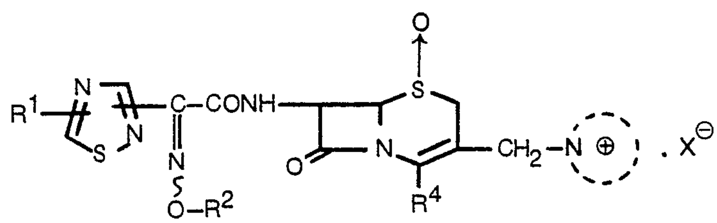

dans laquelle $R^1$, $R^2$, $R^4$, X et le groupe répondant à la formule:

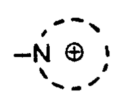

sont chacun tels que définis ci-dessus.

10. Composition pharmaceutique antibactérienne comprenant un composé répondant à la revendication 1 non protégé en association avec un support ou excipient pharmaceutiquement acceptable, pratiquement non toxique.

11. Composé non protégé tel que défini selon l'une quelconque des revendications 1 à 6 pour l'utilisation comme produit pharmaceutique.

12. Composé non protégé tel que défini dans l'une quelconque des revendications 1 à 6 pour l'utilisation dans le traitement de maladies infectieuses.